(19) Europäisches Patentamt
European Patent Office
Office européen des brevets

(11) **EP 2 855 603 B1**

(12) **EUROPEAN PATENT SPECIFICATION**

(45) Date of publication and mention
of the grant of the patent:
**28.09.2022 Bulletin 2022/39**

(21) Application number: **13742294.5**

(22) Date of filing: **24.05.2013**

(51) International Patent Classification (IPC):
***C09D 5/00*** *(2006.01)* ***A61B 17/30*** *(2006.01)*

(52) Cooperative Patent Classification (CPC):
**G02C 7/049; A61B 17/30; C09D 5/00;**
A61B 2017/00853

(86) International application number:
**PCT/US2013/042771**

(87) International publication number:
**WO 2013/177579 (28.11.2013 Gazette 2013/48)**

(54) **MEDICAL DEVICE OR MEDICAL IMPLEMENT**

MEDIZINISCHES GERÄT ODER MEDIZINISCHE VORRICHTUNG

APPAREIL MÉDICAL OU DISPOSITIF MÉDICAL

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **24.05.2012 US 201261651541 P
24.05.2012 US 201261651542 P
24.05.2012 US 201261651543 P**

(43) Date of publication of application:
**08.04.2015 Bulletin 2015/15**

(73) Proprietor: **Massachusetts Institute of Technology
Cambridge, MA 02139 (US)**

(72) Inventors:
• **SMITH, J. David
Arlington, MA 02474 (US)**
• **DHIMAN, Rajeev
Glastonbury
CT06033 (US)**
• **PAXSON, Adam T.
Cambridge
MA 02139 (US)**
• **LOVE, Christohpher J.
Atlantis
FL 33462 (US)**
• **SOLOMON, Brian R.
Rockville
MD 20878 (US)**
• **VARANASI, Kripa K.
MA 02420 (US)**

(74) Representative: **Hoffmann Eitle
Patent- und Rechtsanwälte PartmbB
Arabellastraße 30
81925 München (DE)**

(56) References cited:
**WO-A1-03/071275        WO-A1-2010/129807
WO-A1-2013/130118      WO-A1-2013/141888
WO-A2-02/062568        WO-A2-2006/017009
WO-A2-2013/022467      US-A1- 2004 026 832**

• **TAK-SING WONG ET AL: "Bioinspired
self-repairing slippery surfaces with
pressure-stable omniphobicity", NATURE, vol.
477, no. 7365, 1 January 2011 (2011-01-01), pages
443-447, XP55025132, ISSN: 0028-0836, DOI:
10.1038/nature10447**

## Description

Technical Field

This invention relates generally to liquid-impregnated surfaces. More particularly, in certain embodiments, the invention relates to devices and implements with liquid-impregnated surfaces.

Background

The advent of micro/nano-engineered surfaces in the last decade has opened up new techniques for enhancing a wide variety of physical phenomena in thermofluids sciences. For example, the use of micro/nano surface textures has provided nonwetting surfaces capable of achieving less viscous drag, reduced adhesion to ice and other materials, self-cleaning, and water repellency. These improvements result generally from diminished contact (i.e., less wetting) between the solid surfaces and adjacent liquids.

WO 03/071275 and WO 2010/129807 disclose medical devices or implements having surfaces provided with hydrophilic micro-scale solid features.

Liquid-impregnated surfaces are described in U.S. Patent Application No. 13/302,356, published as US 2013/0032316, entitled, "Liquid-Impregnated Surfaces, Methods of Making, and Devices Incorporating the Same," by Smith et al.; U.S. Patent Application No. 13/517,552, entitled, "Self-Lubricating Surfaces for Food Packaging and Food Processing Equipment," by Smith et al.; and U.S. Provisional Patent Application No. 61/827,444, filed May 24, 2013, entitled, "Apparatus and Methods Employing Liquid-Impregnated Surfaces," by Smith et al.,

There is a need for conduits (e.g., tubes, pipes, channels, vessels, etc.) having low resistance to flow, passage, or removal of material through, into, or out of the conduit; contact lenses with high lubricity to eye tissue and/or eye fluid, for increased comfort, reduced nucleation, and improved resistance to protein build-up and contamination; and devices and implements with high lubricity to flesh (or biological fluid) and/or inhibited nucleation on the surface of the device/implement.

Summary of the Invention

The present invention is defined by the claims. The following information is useful for understanding the invention.

In one aspect of the invention, in some implementations, conduits are provided for conveying fluids and/or solids, the conduits having an interior surface that provide a high-slip boundary condition, thereby facilitating the flow of material therethrough.

In one aspect, the invention provides conduit for conveying fluids and/or solids, the conduit having an interior surface comprising an impregnating liquid and a plurality of micro-scale and/or nano-scale solid features spaced sufficiently close to stably contain the impregnating liquid therebetween. In some implementations, the impregnating liquid fills spaces between the solid features. The interior surface may stably contain the impregnating liquid between the solid features. The impregnating liquid may be substantially held in place between the plurality of solid features regardless of orientation of the interior surface and regardless of flow, passage, or removal of fluids and/or solids through, into, or out of the conduit. The interior surface may be configured to provide a high-slip boundary condition at the interior surface, thereby facilitating the flow, passage, or removal of fluids and/or solids through, into, or out of the conduit.

In some implementations, the conduit is a tube, pipe, or channel. In some implementations, the conduit is a nozzle. In some implementations, the conduit is a mold (e.g., part of an injection molding apparatus) or is part of an extruder. In some implementations, the conduit is a canister or vessel.

In some implementations, the conduit comprises a reservoir for containing liquid for replenishing impregnating liquid lost from the liquid-impregnated surface. In some implementations, the device includes a conduit and a reservoir containing liquid for replenishing impregnating liquid lost from the liquid-impregnated surface.

In some implementations, the impregnating liquid includes at least one member selected from the group consisting of ethyl oleate, an ester, a fatty acid, a fatty acid derivative, a vegetable oil (e.g., olive oil, light olive oil, corn oil, soybean oil, rapeseed oil, linseed oil, grapeseed oil, flaxseed oil, canola oil, peanut oil, safflower oil, sunflower oil), phenyl isothiocyanate (phenyl mustard oil), a terpene, bromobenzene, iodobenzene, o-bromotoluene, alpha-chloronaphthalene, alpha-bromonaphthalene, acetylene tetrabromide, 1-butyl-3-methylimidazolium bis(trifluoromethylsulfonyl) imide (BMIm), tribromohydrin (1,2,3-tribromopropane), ethylene dibromide, carbon disulfide, bromoform, methylene iodide (diiodomethane), stanolax, Squibb's liquid petrolatum, p-bromotoluene, monobromobenzene, perchloroethylene, carbon disulfide, phenyl mustard oil, monoiodobenzene, alpha-monochloro-naphthalene, acetylene tetrabromide, aniline, butyl alcohol, isoamyl alcohol, n-heptyl alcohol, cresol, oleic acid, linoleic acid, amyl phthalate, silicone oil, a perfluorocarbon liquid, a perfluoroFluorinated vaccum oil (such as Krytox 1506 or Fromblin 06/6), a fluorinated coolant (e.g., perfluoro-tripentylamine sold as FC-70, manufactured by 3M), an ionic liquid, a fluorinated ionic liquid that is immiscible with water, a silicone oil comprising PDMS, a fluorinated silicone oil, a liquid metal, an eletro-rheological fluid, a magneto-rheological fluid, a ferrofluid, a dielectric liquid, a hydrocarbon liquid, a fluorocarbon liquid, a refrigerant, a vacuum oil, a phase-change material, a semi-liquid, grease, synovial fluid, and a bodily fluid.

In some implementations, the solid features comprise one or more members selected from the group consisting of wax, carnauba wax, beeswax, candelilla wax, zein (from corn), dextrin, cellulose ether, hydroxyethyl cellulose, hydroxypropyl cellulose (HPC), hydroxyethyl methyl cellulose, hydroxypropyl methyl cellulose (HPMC), ethyl hydroxyethyl cellulose, insoluble fiber, purified wood cellulose, micro-crystalline cellulose, kaolinite (clay mineral), Japan wax, pulp (e.g., spongy part of plant stems), ferric oxide, iron oxide, sodium formate, sodium oleate, sodium palmitate, sodium sulfate, silica, glass, a metal, a polymer (e.g., polytetrafluoroethylene, fluoroacrylate, fluoroeurathane, fluorosilicone, fluorosilane, modified carbonate, chlorosilanes, silicone, polydimethylsiloxane (PDMS)), a ceramic solid, a fluorinated solid, an intermetallic solid, a composite solid, PDMS, cyclic olefin polymer, polypropylene, PVC, PET, HDPE, polyimide, PMMA, glass, Perspex, Plexiglass, Polymacon, a hydrocarbon (e.g., an alkane, a fluoropolymer, teflon, trichloro(1H,1H,2H,2H-perfluorooctyl)silane (TCS), octadecyltrichlorosilane (OTS), heptadecafluoro- 1,1,2,2-tetrahydrodecyltrichlorosilane, fluoroPOSS), a ceramic (e.g., titanium carbide, titanium nitride, chromium nitride, boron nitride, chromium carbide, molybdenum carbide, titanium carbonitride, electroless nickel, zirconium nitride, fluorinated silicon dioxide, titanium dioxide, tantalum oxide, tantalum nitride, diamond-like carbon, fluorinated diamond-like carbon), an intermetallic compound (e.g., nickel aluminide and titanium aluminide), and a composite.

According to the invention, the solid features comprise particles having an average dimension in a rage of 1 micron to 50 microns (e.g., 5 microns to 50 microns). In some implementations, the particles are arranged with average spacing of about 1 microns to about 30 microns (e.g., 10 microns to 30 microns) between adjacent particles or clusters of particles. In some implementations, the particles are spray-deposited.

In some implementations, the solid features comprise or define at least one member selected from the group consisting of particles, amorphous particles, substantially spherical particles, posts, nanoneedles, microneedles, nanograss, micrograss, pores, cavities, wells, interconnected pores, interconnected cavities, grooves, and ridges.

In some implementations, the impregnating liquid comprises an additive to prevent or reduce evaporation of the impregnating liquid.

In another aspect of the invention, in some implementations, two parts are configured to come into contact with each other when the apparatus is in operation, wherein one of or each of the two parts includes a surface with an impregnating liquid and a plurality of micro-scale and/or nano-scale solid features spaced sufficiently close to stably contain the impregnating liquid therebetween. In some implementations, for each of the surfaces, the impregnating liquid fills spaces between the solid features and for each of the surfaces, the surface stably contains the impregnating liquid between the solid features. In some implementations, for each of said surfaces, the impregnating liquid is substantially held in place between the plurality of solid features regardless of orientation of the surfaces and regardless of contact made between the surfaces (e.g., where the apparatus is configured to cause adhesion normal to the surface; e.g., where the apparatus is configured to have low static coefficient of friction; or e.g., where the apparatus is configured to cause adhesion normal to the surface and have low static coefficient of friction). In some implementations, the apparatus is a bearing, a track, or a hinge.

In another aspect of the invention, in some implementations, the disclosed technology is used with an apparatus for capturing solid particulate from air or other gas. The apparatus includes a surface comprising an impregnating liquid and a plurality of micro-scale and/or nano-scale solid features spaced sufficiently close to stably contain the impregnating liquid therebetween. The impregnating liquid fills spaces between the solid features and the surface stably contains the impregnating liquid between the solid features. In some implementations, the impregnating liquid is substantially held in place between said plurality of solid features regardless of orientation of the surface.

In some implementations, the apparatus is an air filter.

The impregnating liquid may have a high viscosity (e.g., greater than 100 cP, or greater than 1000 cP).

In another aspect of the invention, in some implementations, the disclosed technology is used with an apparatus that includes a surface with a curable impregnating liquid and a plurality of micro-scale and/or nano-scale solid features spaced sufficiently close to stably contain the impregnating liquid therebetween, wherein the impregnating liquid fills spaces between said solid features and the surface stably contains the impregnating liquid between the solid features. In some implementations, the impregnating liquid is substantially held in place between the plurality of solid features regardless of orientation of the surface, and wherein the impregnating liquid can be converted to a solid by curing (e.g., exposure to heat).

In some implementations, the disclosed technology is used with an apparatus (e.g., airplane, boat, torpedo, etc.) that includes a surface configured for reduced drag and the surface includes an impregnating liquid and a plurality of micro-scale and/or nano-scale solid features spaced sufficiently close to stably contain the impregnating liquid therebetween. In some implementations, the impregnating liquid fills spaces between the solid features, wherein the surface stably contains the impregnating liquid between the solid features. In some implementations, the impregnating liquid is substantially held in place between the plurality of solid features regardless of orientation of the surface.

According to the invention, medical devices and implements are provided with liquid-impregnated surfaces for enhanced lubricity to flesh (or biological fluid) and/or inhibited nucleation on the surface of the device/implement.

The invention provides a medical device or medical implement with high lubricity to flesh (or biological fluid) and/or inhibited nucleation on its surface, the device or implement includes a surface comprising an impregnating liquid and a plurality of micro-scale and/or nano-scale solid features spaced sufficiently close to stably contain the impregnating liquid therebetween. In certain embodiments, the impregnating liquid fills spaces between said solid features and the surface stably contains the impregnating liquid between the solid features. In certain embodiments, the impregnating liquid is substantially held in place between the solid features regardless of orientation of the surface.

The solid features comprise particles having an average dimension in a range of 1 micron to 50 microns (e.g., 5 microns to 50 microns). The particles may be arranged with average spacing of about 1 micron to about 30 microns between adjacent particles or clusters of particles (e.g., 10 microns to 30 microns). The particles may be spray-deposited.

In certain embodiments, the impregnating liquid includes at least one member selected from the group consisting of ethyl oleate, an ester, a fatty acid, a fatty acid derivative, a vegetable oil (e.g., olive oil, light olive oil, corn oil, soybean oil, rapeseed oil, linseed oil, grapeseed oil, flaxseed oil, canola oil, peanut oil, safflower oil, sunflower oil), a terpene, phenyl isothiocyanate (phenyl mustard oil), bromobenzene, iodobenzene, o-bromotoluene, alpha-chloronaphthalene, alpha-bromonaphthalene, acetylene tetrabromide, 1-butyl-3-methylimidazolium bis(trifluoromethylsulfonyl) imide (BMlm), tribromohydrin (1,2,3-tribromopropane), ethylene dibromide, carbon disulfide, bromoform, methylene iodide (diiodomethane), stanolax, Squibb's liquid petrolatum, p-bromotoluene, monobromobenzene, perchloroethylene, carbon disulfide, phenyl mustard oil, monoiodobenzene, alpha-monochloro-naphthalene, acetylene tetrabromide, aniline, butyl alcohol, isoamyl alcohol, n-heptyl alcohol, cresol, oleic acid, linoleic acid, and amyl phthalate.

In certain embodiments, the solid features include one or more members selected from the group consisting of wax, carnauba wax, beeswax, candelilla wax, zein (from corn), dextrin, cellulose ether, hydroxyethyl cellulose, hydroxypropyl cellulose (HPC), hydroxyethyl methyl cellulose, hydroxypropyl methyl cellulose (HPMC), ethyl hydroxyethyl cellulose, insoluble fiber, purified wood cellulose, micro-crystalline cellulose, kaolinite (clay mineral), Japan wax, pulp (e.g., spongy part of plant stems), ferric oxide, iron oxide, sodium formate, sodium oleate, sodium palmitate, sodium sulfate, a metal, a polymer, a ceramic solid, a fluorinated solid, an intermetallic solid, and a composite solid PDMS, cyclic olefin polymer, polypropylene, PVC, PET, HDPE, polyimide, PMMA, glass, Perspex, Plexiglass, Polymacon.

The impregnating liquid may include an additive to prevent or reduce evaporation of the impregnating liquid. The medical device or medical implement may be a member selected from the group consisting of braces, dentures, a retainer, orthodonture, a bridge, an implant, a tooth/teeth mold, a prosthesis, an artificial organ, an artificial artery, a stent, a syringe, a lining (e.g., lining for artery walls to prevent plaque formation), an IV tube, an IV bag, a colostomy bag, a surgical instrument, a bandage, and a blood pump.

The medical device or medical implement may be a blood pump or part thereof. The surface may be configured to provide reduction of shear forces to prevent damage to cells and/or other biological structures in blood or other biological fluids being pumped thereby or therethrough. The medical device or medical implement may be a member selected from the group consisting of a pill, capsule (e.g., single-piece or two-piece), tablet, gel cap, and suppository.

The medical device or medical implement may be a member selected from the group consisting of a micropoipette, a small volume container of biological material, a human serum container, a pipette, a pipette tip, a microfluidic device, a dialysis machine, a tube, an endoscope, an intubation device, a syringe, a stent, a catheter, and a tracheotomy tube.

The medical device or medical implement may be a member selected from the group consisting of a glove, bandage, adhesive strip, drug release patch, and condom. The impregnating liquid may be an antiseptic and/or an antibacterial. The impregnating liquid may be curable and can be converted to a solid by curing (e.g., exposure to heat).

In one aspect of the invention, in some implementations, contact lenses are provided with liquid-impregnated surfaces for enhanced lubricity to eye tissue and/or eye fluid, for increased comfort, reduced nucleation, and improved resistance to protein build-up and contamination.

In one aspect, the invention provides a contact lens with high lubricity to eye tissue/fluid and/or with inhibited nucleation on its surface, the contact lens includes a surface textured to form a matrix of micro-scale and/or nano-scale solid (e.g., gel) features spaced sufficiently close to stably contain an impregnating liquid therebetween. The impregnating liquid fills spaces between the solid features. The surface may stably contain the impregnating liquid between the solid features. The impregnating liquid may be substantially held in place between the solid features regardless of orientation of the surface and despite contact with the eye tissue during normal wear, insertion, and removal of the contact lens.

In some implementations, the features define pores or cavities and the impregnating liquid fills the pores or cavities. The matrix may have a feature-to-feature spacing from about 1 micrometer to about 100 micrometers. The matrix has a feature-to-feature spacing from about 5 nanometers to about 1 micrometer. The surface is laser-etched to form said matrix of solid features. The impregnating liquid is substantially immiscible with eye fluid (e.g., substantially immiscible with a saline solution).

The solid features and/or the material of the lens itself may include one or more members selected from the group consisting of polymer, hydrogel, polyimide, polymacon, silicone hydrogel, polymethyl methacrylate (PMMA or Perspex/Plexiglas), and glass.

The solid features may include one or more members selected from the group consisting of wax, carnauba wax, beeswax, candelilla wax, zein (from corn), dextrin, cellulose ether, hydroxyethyl cellulose, hydroxypropyl cellulose (HPC), hydroxyethyl methyl cellulose, hydroxypropyl methyl cellulose (HPMC), ethyl hydroxyethyl cellulose, insoluble fiber, purified wood cellulose, micro-crystalline cellulose, kaolinite (clay mineral), Japan wax, pulp (e.g., spongy part of plant stems), ferric oxide, iron oxide, sodium formate, sodium oleate, sodium palmitate, sodium sulfate, silica, a metal, a polymer, a ceramic solid, a fluorinated solid, an intermetallic solid, and a composite solid, PDMS, cyclic olefin polymer, polypropylene, PVC, PET, and HDPE.

The impregnating liquid may include at least one member selected from the group consisting of ethyl oleate, an ester, a fatty acid, a fatty acid derivative, a terpene, an oil, tetrachloroethylene (perchloroethylene), phenyl isothiocyanate, bromobenzene, iodobenzene, o-bromotoluene, alpha-chloronaphthalene, alpha-bromonaphthalene, acetylene tetrabromide, 1-butyl-3-methylimidazolium bis(trifluoromethylsulfonyl) imide (BMIm), tribromohydrin (1,2,3-tribromopropane), ethylene dibromide, carbon disulfide, bromoform, methylene iodide (diiodomethane), stanolax, liquid petrolatum, p-bromotoluene, monobromobenzene, perchloroethylene, carbon disulfide, phenyl mustard oil, monoiodobenzene, alpha-monochloro-naphthalene, acetylene tetrabromide, aniline, butyl alcohol, isoamyl alcohol, n-heptyl alcohol, cresol, oleic acid, linoleic acid, and amyl phthalate.

In some implementations, the impregnating liquid includes a medication for delivery onto the eye.

In some implementations, the impregnating liquid is colored (e.g., for colored contact lenses).

In some implementations, the impregnating liquid forms a liquid layer extending above the top of the solid features of the surface while at equilibrium or substantially at equilibrium.

In some implementations, the liquid layer extends above the top of the solid features by at least about 5 nm.

In some implementations, the impregnating liquid includes a drug (e.g. antiseptic and/or an antibacterial, or bioactive components (including but not limited to drugs, vitamins, minerals, proteins).

In some implementations, one or both of the following holds: (i) $0 < \phi \leq 0.25$, where $\phi$ is a representative fraction of the projected surface area of the liquid-impregnated surface corresponding to non-submerged solid at equilibrium; and (ii) $S_{ow(a)} < 0$, where $S_{ow(a)}$ is spreading coefficient, defined as $\gamma_{wa} - \gamma_{wo} - \gamma_{oa}$, where $\gamma$ is the interfacial tension between the two phases designated by subscripts w, a, and o, where w is water, a is air, and o is the impregnating liquid.

In some implementations, one or both of the following holds: (i) $0 < \phi \leq 0.25$, where $\phi$ is a representative fraction of the projected surface area of the liquid-impregnated surface corresponding to non-submerged solid at equilibrium; and (ii) $S_{ow(a)} < 0$, where $S_{ow(a)}$ is spreading coefficient, defined as $\gamma_{wa} - \gamma_{wo} - \gamma_{oa}$, where $\gamma$ is the interfacial tension between the two phases designated by subscripts w, a, and o, where w is water, a is air, and o is the impregnating liquid. In some implementations, $0 < \phi \leq 0.25$. In some implementations, $0 < \phi \leq 0.10$. In some implementations, $0.01 < \phi \leq 0.25$. In some implementations, $0.01 < \phi \leq 0.10$. In some implementations, $S_{ow(a)} < 0$.

In some implementations, one or both of the following holds: (i) $\theta_{os(w),receding} = 0$; and (ii) $\theta_{os(a),receding} = 0$ and $\theta_{os(w),receding} = 0$, where $\theta_{os(w),receding}$ is receding contact angle of the impregnating liquid (e.g., oil, subscript 'o') on the surface (subscript 's') in the presence of water (subscript 'w'), and where $\theta_{os(a),receding}$ is receding contact angle of the impregnating liquid (e.g., oil, subscript 'o') on the surface (subscript 's') in the presence of air (subscript 'a').

Brief Description of the Drawings

The objects and features of the invention can be better understood with reference to the drawing described below, and the claims.

FIG. 1 illustrates a schematic cross-sectional and corresponding top view of a liquid-impregnated surface that are partially submerged;

FIGS. 2A and 2B demonstrate the effectiveness of liquid-impregnated surface coatings on tweezers to shed off blood;

FIGS. 3A and 3B demonstrate that liquid-impregnated surface pills slide more easily on animal tissue than uncoated pills;

FIGS. 4A and 4B demonstrate that animal flesh slides more easily on liquid-impregnated surfaces than on uncoated surfaces;

FIGS. 5A though 5D illustrate a mold-release experiment using concrete and a liquid-impregnated surface coated mold;

FIG. 6 illustrates a sold-to-solid adhesion experiment for determining the adhesion strength of liquid-impregnated surfaces;

FIGS. 7A through 7F illustrate the effect of liquid-impregnated surfaces on tubes, pipes, channels, or other similar items;

FIGS. 8A and 8B illustrates the results from an injection molding experiment;

FIGS. 9A through 9D illustrate a dust capture experiment on liquid-impregnated surfaces;

FIG. 10A through 10E illustrates a conduit experiment for demonstrating the properties of a liquid-impregnated surface on a surface that receives a viscous material;

FIGS. 11A through 11D illustrate an experiment conducted to measure the drag on a metal sphere; and

FIG. 12 illustrates the wetting behavior of a sphere with a liquid-impregnated surface, a sphere with a textured outer surface, an a sphere with a smooth surface.

FIGS. 13A and 13B illustrates the appearance and transparency of a liquid-impregnated surface coated contact lens with respect to an uncoated contact lens.

Detailed Description

It is contemplated that compositions, mixtures, systems, devices, methods, and processes of the claimed invention encompass variations and adaptations developed using information from the embodiments described herein. Adaptation and/or modification of the compositions, mixtures, systems, devices, methods, and processes described herein may be performed by those of ordinary skill in the relevant art.

Throughout the description, where articles, devices, apparatus and systems are described as having, including, or comprising specific components, or where processes and methods are described as having, including, or comprising specific steps, it is contemplated that, additionally, there are articles, devices, apparatus and systems of the present invention that consist essentially of, or consist of, the recited components, and that there are processes and methods according to the present invention that consist essentially of, or consist of, the recited processing steps.

Similarly, where articles, devices, mixtures, apparatus and compositions are described as having, including, or comprising specific compounds and/or materials, it is contemplated that, additionally, there are articles, devices, mixtures, apparatus and compositions of the present invention that consist essentially of, or consist of, the recited compounds and/or materials.

It should be understood that the order of steps or order for performing certain actions is immaterial so long as the invention remains operable. Moreover, two or more steps or actions may be conducted simultaneously.

The mention herein of any publication, for example, in the Background section, is not an admission that the publication serves as prior art with respect to any of the claims presented herein. The Background section is presented for purposes of clarity and is not meant as a description of prior art with respect to any claim.

Described herein are surfaces comprising an impregnating liquid and a plurality of micro-scale and/or nano-scale solid features spaced sufficiently close to stably contain the impregnating liquid therebetween, wherein the impregnating liquid fills spaces between the solid features, wherein the interior surface stably contains the impregnating liquid between the solid features, and wherein the impregnating liquid is substantially held in place between the plurality of solid features.

In certain embodiments, the solid features may be part of the surface itself (e.g., the surface may be etched or otherwise textured to create the solid features), or the solid features may be applied to the surface. In certain embodiments, the solid features include an intrinsically hydrophobic, oleophobic, and/or metallophobic material or coating. For example, the solid features may be made of: hydrocarbons, such as alkanes, and fluoropolymers, such as teflon, trichloro(1H,1H,2H,2H-perfluorooctyl)silane (TCS), octadecyltrichlorosilane (OTS), heptadecafluoro- 1,1,2,2-tetrahydrodecyltrichlorosilane, fluoroPOSS, and/or other fluoropolymers. Additional possible materials include: ceramics, polymeric materials, fluorinated materials, intermetallic compounds, and composite materials. Polymeric materials may include, for example, polytetrafluoroethylene, fluoroacrylate, fluoroeurathane, fluorosilicone, fluorosilane, modified carbonate, chlorosilanes, silicone, polydimethylsiloxane (PDMS), and/or combinations thereof. Ceramics may include, for example, titanium carbide, titanium nitride, chromium nitride, boron nitride, chromium carbide, molybdenum carbide, titanium carbonitride, electroless nickel, zirconium nitride, fluorinated silicon dioxide, titanium dioxide, tantalum oxide, tantalum nitride, diamond-like carbon, fluorinated diamond-like carbon, and/or combinations thereof. Intermetallic compounds may include, for example, nickel aluminide, titanium aluminide, and/or combinations thereof.

The solid features of a liquid-impregnated surface may form physical textures or surface roughness. The textures may be random, including fractal, or patterned. In certain embodiments, the textures are micro-scale or nano-scale features. For example, the textures may have a length scale L (e.g., an average pore diameter, or an average protrusion height) that is less than about 100 microns, less than about 10 microns, less than about 1 micron, less than about 0.1 microns, or less than about 0.01 microns. In certain embodiments, the texture includes posts or other protrusions, such as spherical or hemispherical protrusions. Rounded protrusions may be preferable to avoid sharp solid edges and minimize pinning of liquid edges. The texture may be introduced to the surface using any conventional method, including mechanical and/or chemical methods.

The solid features include particles. In certain embodiments, the particles have an average characteristic dimension in a range, for example, of about 10 microns to about 50 microns. In certain embodiments, the characteristic dimension is a diameter (e.g., for roughly spherical particles), a length (e.g., for roughly rod-shaped particles), a thickness, a depth, or a height. In certain embodiments, the particles include insoluble fibers, purified wood cellulose, micro-crystalline cellulose, oat bran fiber, kaolinite (clay mineral), Japan wax (obtained from berries), pulp (spongy part of plant stems), ferric oxide, iron oxide, sodium formate, sodium oleate, sodium palmitate, sodium sulfate, wax, carnauba wax, beeswax, candelilla wax, zein (from corn), dextrin, cellulose ether, Hydroxyethyl cellulose, Hydroxypropyl cellulose (HPC), Hydroxyethyl methyl cellulose, Hydroxypropyl methyl cellulose (HPMC), and/or Ethyl hydroxyethyl cellulose. In certain embodiments, the particles include a wax. In certain embodiments, the particles are randomly spaced. In certain embodiments, the particles are arranged with average spacing of about 1 micron to about 500 microns, or from about 5 microns to about 200 microns, or from about 10 microns to about 30 microns between adjacent particles or clusters of particles. In certain embodiments, the particles are spray-deposited (e.g., deposited by aerosol or other spray mechanism).

In the invention, micro-scale features are used. The micro-features comprise particles. Particles can be randomly or uniformly dispersed on a surface. Characteristic spacing between particles can be about 200 $\mu$m, about 100 $\mu$m, about 90 $\mu$m, about 80 $\mu$m, about 70 $\mu$m, about 60 $\mu$m, about 50 $\mu$m, about 40 $\mu$m, about 30 $\mu$m, about 20 $\mu$m, about 10 $\mu$m, about 5 $\mu$m or 1 $\mu$m. In some embodiments, characteristic spacing between particles is in a range of 100 $\mu$m to 1 $\mu$m, 50 $\mu$m to 20 $\mu$m, or 40 $\mu$m to 30 $\mu$m. In some embodiments, characteristic spacing between particles is in a range of 100 $\mu$m to 80 $\mu$m, 80 $\mu$m to 50 $\mu$m, 50 $\mu$m to 30 $\mu$m or 30 $\mu$m to 10 $\mu$m. In some embodiments, characteristic spacing between particles is in a range of any two values above.

Particles can have an average dimension of about 50 $\mu$m, about 40 $\mu$m, about 30 $\mu$m, about 20 $\mu$m, about 10 $\mu$m, about 5 $\mu$m or 1 $\mu$m. In some embodiments, an average dimension of particles is in a range of 50 $\mu$m to 10 $\mu$m, or 30 $\mu$m to 20 $\mu$m. In some embodiments, an average dimension of particles is in a range of 50 $\mu$m to 30 $\mu$m, or 30 $\mu$m to 10 $\mu$m. In some embodiments, an average dimension of particles is in a range of any two values above.

In some embodiments, particles are porous. Characteristic pore size (e.g., pore widths or lengths) of particles can be about 5000 nm, about 3000 nm, about 2000 nm, about 1000 nm, about 500 nm, about 400 nm, about 300 nm, about 200 nm, about 100 nm, about 80 nm, about 50, about 10 nm. In some embodiments, characteristic pore size is in a range of 200 nm to 2 $\mu$m or 100 nm to 1 $\mu$m. In some embodiments, characteristic pore size is in a range of any two values above.

The impregnating liquid of a liquid-impregnating surface may be oil-based or water-based (i.e., aqueous). The liquid may be chosen for a given application based on its properties. In certain embodiments, the impregnating liquid is an ionic liquid (e.g., BMI-IM). Other examples of possible impregnating liquids include hexadecane, vacuum pump oils (e.g., FOMBLIN® 06/6, KRYTOX® 1506) silicon oils (e.g., 10 cSt or 1000 cSt), fluorocarbons (e.g., perfluoro-tripentylamine, FC-70), shear-thinning fluids, shear-thickening fluids, liquid polymers, dissolved polymers, viscoelastic fluids, and/or liquid fluoroPOSS. In one embodiment, the impregnating liquid is made shear thickening with the introduction of nano particles. A shear-thickening impregnating liquid may be desirable for preventing impalement and resisting impact from impinging liquids, for example. To minimize evaporation of the impregnating liquid from the surface, it may be desirable to use an impregnating liquid that has a low vapor pressure (e.g., less than 0.1 mmHg, less than 0.001 mmHg, less than 0.00001 mmHg, or less than 0.000001 mmHg). In certain embodiments, the impregnating liquid has a freezing point of less than -20 °C, less than -40 °C, or about -60 °C. In certain embodiments, the surface tension of the impregnating liquid is about 15 mN/m, about 20 mN/m, or about 40 mN/m. In certain embodiments, the viscosity of the impregnating liquid is from about 10 cSt to about 1000 cSt.

The impregnating liquid may be introduced to the surface using a conventional technique for applying a liquid to a solid. In certain embodiments, a coating process, such as a dip coating, blade coating, or roller coating, is used to apply the impregnating liquid. Alternatively, the impregnating liquid may be introduced and/or replenished by liquid materials flowing past the surface. In preferred embodiments, after the impregnating liquid has been applied, capillary forces hold the liquid in place.

In certain embodiments, a texture may be applied to a substrate to form a surface with solid features. Applying the texture may include: exposing the substrate to a solvent (e.g., solvent-induced crystallization), extruding or blow-molding a mixture of materials, roughening the substrate with mechanical action (e.g., tumbling with an abrasive), spray-coating, polymer spinning, depositing particles from solution (e.g., layer-by-layer deposition and/or evaporating away liquid from a liquid and particle suspension), extruding or blow-molding a foam or foam-forming material (e.g., a polyurethane foam), depositing a polymer from a solution, extruding or blow-molding a material that expands upon cooling to leave a wrinkled or textured surface, applying a layer of material onto a surface that is under tension or compression, performing nonsolvent induced phase separation of a polymer to obtain a porous structure, performing micro-contact printing, performing laser rastering, performing nucleation of the solid texture out of vapor (e.g., desublimation), performing anodization, milling, machining, knurling, e-beam milling, performing thermal or chemical oxidation, and/or performing chemical vapor deposition. In certain embodiments, applying the texture to the substrate includes spraying a mixture of edible particles onto the substrate. In certain embodiments, impregnating the matrix of features with the liquid includes: spraying the encapsulating liquid onto the matrix of features, brushing the liquid onto the matrix of features, submerging the matrix of features in the liquid, spinning the matrix of features, condensing the liquid onto the matrix of features, depositing a solution comprising the liquid and one or more volatile liquids, and/or spreading the liquid over the surface with a second immiscible liquid. In certain embodiments, the liquid is mixed with a solvent and then sprayed, because the solvent will reduce the liquid viscosity, allowing it to spray more easily and more uniformly. Then, the solvent will dry out of the coating. In certain embodiments, the method further includes chemically modifying the substrate prior to applying the texture to the substrate and/or chemically modifying the solid features of the texture. For example, the method may include chemically modifying with a material having contact angle with water of greater than 70 degrees (e.g., hydrophobic material). The modification may be conducted, for example, after the texture is applied, or may be applied to particles prior to their application to the substrate. In certain embodiments, impregnating the matrix of features includes removing excess liquid from the matrix of features. In certain embodiments, removing the excess liquid includes: using a second immiscible liquid to carry away the excess liquid, using mechanical action to remove the excess liquid, absorbing the excess liquid using a porous material, and/or draining the excess liquid off of the matrix of features using gravity or centrifugal forces.

Liquid-impregnated surfaces are useful for reducing viscous drag between a solid surface and a flowing liquid. In general, the viscous drag or shear stress exerted by a liquid flowing over a solid surface is proportional to the viscosity of the liquid and the shear rate adjacent to the surface. A traditional assumption is that liquid molecules in contact with the solid surface stick to the surface, in a so-called "no-slip" boundary condition. While some slippage may occur between the liquid and the surface, the no-slip boundary condition is a useful assumption for most applications. In certain embodiments, liquid-impregnated surfaces are desirable as they induce a large amount of slip at the solid surface. Drag reductions of as much as 40% may be achieved due to this slippage.

In certain embodiments, impregnating a liquid within the textures of a liquid-impregnated surface prevents or reduces nucleation in these regions. The reduction in nucleation is enhanced where liquid covers the tops of the solid features of the liquid-impregnated surface. Furthermore, in certain embodiments, liquid-impregnated surfaces have low roll-off angles (i.e., the angle or slope of a surface at which a droplet in contact with the surface will begin to roll or slide off the surface). The low roll-off angles associated with liquid-impregnated surfaces allow droplets in contact with the surface to easily roll off the surface before the liquid can accumulate on the surface. In certain embodiments, liquid-impregnated surfaces are used to provide hydrate-phobicity, thereby preventing or minimizing the formation of hydrates. In certain embodiments, liquid-impregnated surfaces are used to provide salt-phobicity, thereby preventing or minimizing the formation of salts or mineral scale.

In certain embodiments, liquid-impregnated surfaces are used to reduce viscous drag between a solid surface and a flowing liquid. In certain embodiments, a liquid-impregnated surface is used to provide lubrication between the liquid-impregnated surface and a substance in contact with the surface (or the surface itself, where one liquid-impregnated surface rubs against another liquid-impregnated surface, or parts of the liquid-impregnated surface rub against each other). For example, liquid-impregnated surfaces may provide significant slip/lubrication advantages when in contact with a substance that is a non-Newtonian material, a Bingham plastic, a thixotropic fluid, and/or a shear-thickening substance.

Liquid-impregnated surfaces may also provide anti-fouling and/or self-cleaning. Liquid-impregnated surfaces may also be used to promote the condensation of moisture.

As used herein, emerged area fraction $\phi$ is defined as a representative fraction of the projected surface area of (a representative fraction of) the liquid-impregnated surface corresponding to non-submerged solid at equilibrium (or pseudo-equilibrium). The term "equilibrium" as used herein refers to the condition in which the average thickness of the impregnating film does not substantially change over time due to drainage by gravity when the substrate is held away from horizontal, and where evaporation is negligible (e.g., if the liquid impregnated liquid were to be placed in an environment saturated with the vapor of that impregnated liquid). Similarly, the term "pseudo-equilibrium" as used herein refers to the same condition except that evaporation may occur.

In general, a "representative fraction" of a surface refers to a portion of the surface with a sufficient number of solid features thereupon such that the portion is reasonably representative of the whole surface. In certain embodiments, a "representative fraction" is at least a tenth of the whole surface.

In certain embodiments, $\phi$ is zero (there is a layer of liquid over the top of the solid features which may be, for example, at least 1 nm, at least 5 nm, at least 10 nm, or at least 100 nm in thickness). In certain embodiments, $\phi$ is less than 0.30, 0.25, 0.20, 0.15, 0.10, 0.05, 0.01, or 0.005. In certain embodiments, $\phi$ is greater than 0.001, 0.005, 0.01, 0.05, 0.10, 0.15, or 0.20. In certain embodiments, $\phi$ is in a range of about 0 and about 0.25. In certain embodiments, $\phi$ is in a range of about 0 and about 0.01. In certain embodiments, $\phi$ is in a range of about 0.001 and about 0.25. In certain embodiments, $\phi$ is in a range of about 0.001 and about 0.10.

In some embodiments, the liquid-impregnated surface is configured such that cloaking by the impregnating liquid can be either eliminated or induced, according to different embodiments described herein.

As used herein, the spreading coefficient, $S_{ow(a)}$ is defined as $\gamma_{wa} - \gamma_{wo} - \gamma_{oa}$, where $\gamma$ is the interfacial tension between the two phases designated by subscripts w, a, and o, where w is water, a is air, and o is the impregnating liquid. Interfacial tension can be measured using a pendant drop method as described in Stauffer, C. E., "The measurement of surface tension by the pendant drop technique," J. Phys. Chem. 1965, 69, 1933-1938, the text of which is incorporated by reference herein. Exemplary surfaces and its interfacial tension measurements (at approximately 25 °C) are shown in Appendix D, in particular, Table S2.

Without wishing to be bound to any particular theory, impregnating liquids that have $S_{ow(a)}$ less than 0 will not cloak, resulting in no loss of impregnating liquids, whereas impregnating liquids that have $S_{ow(a)}$ greater than 0 will cloak matter (condensed water droplets, bacterial colonies, solid surface) and this may be exploited to prevent corrosion, fouling, etc. In certain embodiments, cloaking is used for preventing vapor-liquid transformation (e.g, water vapor, metallic vapor, etc.). In certain embodiments, cloaking is used for inhibiting liquid-solid formation (e.g., ice, metal, etc.). In certain embodiments, cloaking is used to make reservoirs for carrying the materials, such that independent cloaked materials can be controlled and directed by external means (like electric or magnetic fields).

In certain embodiments, lubricant cloaking is desirable and is used a means for preventing environmental contamination, like a time capsule preserving the contents of the cloaked material. Cloaking can result in encasing of the material thereby cutting its access from the environment. This can be used for transporting materials (such as bioassays) across a length in a way that the material is not contaminated by the environment.

In certain embodiments, the amount of cloaking can be controlled by various lubricant properties such as viscosity, surface tension. Additionally or alternatively, we can control the de-wetting of the cloaked material to release the material. Thus, it is contemplated that a system in which a liquid is dispensed in the lubricating medium at one end, and upon reaching the other end is exposed to environment that causes the lubricant to uncloak.

In some embodiments, an impregnating liquid can be selected to have a $S_{ow(a)}$ less than 0. Exemplary impregnating liquids include, but are not limited to, tetrachloroethylene (perchloroethylene), phenyl isothiocyanate (phenyl mustard oil), bromobenzene, iodobenzene, o-bromotoluene, alpha-chloronaphthalene, alpha-bromonaphthalene, acetylene tetrabromide, 1-butyl-3-methylimidazolium bis(trifluoromethylsulfonyl) imide (BMIm), tribromohydrin (1,2,3-tribromopropane), tetradecane, cyclohexane, ethylene dibromide, carbon disulfide, bromoform, methylene iodide (diiodomethane), stanolax, Squibb's liquid petrolatum, p-bromotoluene, monobromobenzene, perchloroethylene, carbon disulfide, phenyl mustard oil, monoiodobenzene, alpha-monochloro-naphthalene, acetylene tetrabromide, aniline, butyl alcohol, isoamyl alcohol, n-heptyl alcohol, cresol, oleic acid, linoleic acid, amyl phthalate and any combination thereof.

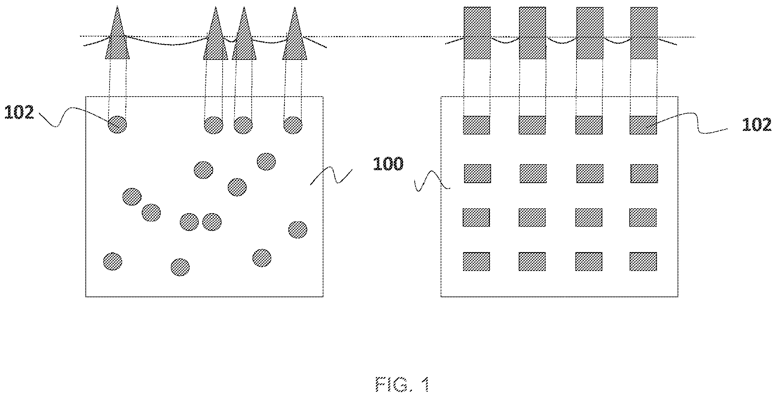

Referring to FIG. 1, a schematic cross-sectional view and the corresponding top view of a liquid-impregnated surface that is partially submerged is shown. The upper left drawing of FIG. 1 shows a cross-sectional view of a row of cone-shaped solid features. The projected surface area of the non-submerged solid 102 is illustrated as shaded areas of the overhead view, while the remaining non-shaded area represents the projected surface area of the submerged liquid-impregnated surface 100. In addition to the projection surface area of this row of solid features, other solid features placed in a semi-random pattern are shown in shade in the overhead view. Similarly, the cross-section view of a row of evenly spaced posts is shown on the right of FIG. 1. Additional rows of well-patterned posts are shown in shade in the overhead view. As demonstrated, in some embodiments, a liquid-impregnated surface includes randomly and/or non-randomly patterned solid features.

In certain embodiments, a material exhibits the nucleation show in FIG. 1 on its surface. The material's surface comprises an array of micro-scale or nano-scale solid features spaced sufficiently close to contain an impregnating liquid in between them. The impregnating liquid fills the spaces between the solid features, and the surface stably holds the impregnating liquid in place in between the solid features regardless of the orientation of the surface. In some implementations, the particles have an average dimension of 5 microns to 50 microns. In some implementations, the particles are arranged with average spacing of about 10 microns to about 30 microns between adjacent particles or clusters of particles.

In certain embodiments, the particles are coated onto the material's surface by spray coating the surface with an impregnating liquid solution. The spray coating may apply a uniform coat of impregnating liquid to the surface of the material. In certain implementations, the impregnating liquid may be spray coated onto the surface of the material in multiple stages. In certain implementations where the impregnating solution is composed of several different solutions, the various constituent solutions of the impregnating liquid may be spray coated onto the target surface in different stages.

The applications of liquid-impregnated surfaces could extend, for example, to a conduit for conveying fluids and/or solids. The conduit may have an interior surface comprising an impregnating liquid and micro-scale and/or nano-scale solid features spaced sufficiently close to stably contain the impregnating liquid therebetween. The impregnating liquid may fill spaces between the solid features. In some implementations, the interior surface stably contains the impregnating liquid between the solid features. The impregnating liquid may be substantially held in place between the of solid features regardless of orientation of the interior surface and regardless of flow, passage, or removal of fluids and/or solids through, into, or out of the conduit. The interior surface may be configured to provide a high-slip boundary condition at the interior surface, thereby facilitating the flow, passage, or removal of fluids and/or solids through, into, or out of the conduit.

In some implementations, the conduit includes a reservoir for containing liquid for replenishing impregnating liquid lost from the liquid-impregnated surface. The reservoir may provide a continuous supplying liquid-impregnated surface solution back to the desired surface. in some implementations, the reservoir is used to replenish impregnating liquid lost from the liquid-impregnated surface.

In some implementations, the impregnating liquid is an ethyl oleate, a fatty acid, a vegetable oil (e.g., olive oil, light olive oil, corn oil, soybean oil, rapeseed oil, linseed oil, grapeseed oil, flaxseed oil, canola oil, peanut oil, safflower oil, sunflower oil), tetrachloroethylene (perchloroethylene), phenyl isothiocyanate (phenyl mustard oil), bromobenzene, iodobenzene, o-bromotoluene, alpha-chloronaphthalene, alpha-bromonaphthalene, acetylene tetrabromide, 1-butyl-3-methylimidazolium bis(trifluoromethylsulfonyl) imide (BMIm), tribromohydrin (1,2,3-tribromopropane), ethylene dibromide, carbon disulfide, bromoform, methylene iodide (diiodomethane), stanolax, Squibb's liquid petrolatum, p-bromotoluene, monobromobenzene, perchloroethylene, carbon disulfide, phenyl mustard oil, monoiodobenzene, alpha-monochloro-naphthalene, acetylene tetrabromide, aniline, butyl alcohol, isoamyl alcohol, n-heptyl alcohol, cresol, oleic acid, linoleic acid, and/or amyl phthalate. The impregnating liquid may include an additive to prevent or reduce evaporation of the impregnating liquid.

In some implementations, the solid features include wax, carnauba wax, beeswax, candelilla wax, zein (from corn), dextrin, cellulose ether, hydroxyethyl cellulose, hydroxypropyl cellulose (HPC), hydroxyethyl methyl cellulose, hydroxypropyl methyl cellulose (HPMC), ethyl hydroxyethyl cellulose, insoluble fiber, purified wood cellulose, micro-crystalline cellulose, kaolinite (clay mineral), Japan wax, pulp (e.g., spongy part of plant stems), ferric oxide, iron oxide, sodium formate, sodium oleate, sodium palmitate, sodium sulfate, a metal, a polymer, a ceramic solid, a fluorinated solid, an intermetallic solid, and/or a composite solid.

The solid features may include particles having an average dimension in a rage of 5 microns to 50 microns. The particles may be arranged with average spacing of about 10 microns to about 30 microns between adjacent particles or clusters of particles. The particles may be spray-deposited.

The solid features may include particles, amorphous particles, substantially spherical particles, posts, nanoneedles, microneedles, nanograss, micrograss, pores, cavities, wells, interconnected pores, and/or interconnected cavities.

The applications of liquid-impregnated surfaces could extend, for example, to an apparatus with two parts configured to come into contact with each other when the apparatus is in operation. Each of the two parts may include a surface comprising an impregnating liquid and micro-scale and/or nano-scale solid features spaced sufficiently close to stably contain the impregnating liquid therebetween. For each of the surfaces, the impregnating liquid fills spaces between said solid features. Each surface may stably contain the impregnating liquid between the solid features. For each of the surfaces, the impregnating liquid is substantially held in place between the solid features regardless of orientation of the surfaces and regardless of contact made between the surfaces. In some implementations, the apparatus is a thrust bearing.

In some implementations, the disclosed technology includes an apparatus for capturing solid particulate from air or other gas. The apparatus may include a surface with an impregnating liquid and a micro-scale and/or nano-scale solid features spaced sufficiently close to stably contain the impregnating liquid therebetween. The impregnating liquid may fill spaces between the solid features. The surface may stably contain the impregnating liquid between the solid features. The impregnating liquid may be substantially held in place between the solid features regardless of orientation of the surface. in some implementations, the apparatus is an air filter. The impregnating liquid has high viscosity (e.g., greater than 1000 cP).

The disclosed technology, in some implementations, may include a surface with a curable impregnating liquid and micro-scale and/or nano-scale solid features spaced sufficiently close to stably contain the impregnating liquid therebetween. The impregnating liquid may fill spaces between the solid features. The surface may stably contain the impregnating liquid between the solid features. The impregnating liquid may be substantially held in place between the solid features regardless of orientation of the surface. The impregnating liquid may be converted to a solid by curing (e.g., exposure to heat).

The applications of liquid-impregnated surfaces could extend, for example, to an apparatus (e.g., vehicle, automobile, airplane, boat, torpedo, missile, etc.) with a surface configured for reduced drag. The surface may include an impregnating liquid and micro-scale and/or nano-scale solid features spaced sufficiently close to stably contain the impregnating liquid therebetween. The impregnating liquid may fill spaces between the solid features. The surface may stably contain the impregnating liquid between the solid features. The impregnating liquid may be substantially held in place between the solid features regardless of orientation of the surface.

The applications of liquid-impregnated surfaces could extend, for example, to in fluid conveyances and manufacturing equipment, systems, and processes, such as those described below. In some implementations, liquid-impregnated surfaces are used for pumping, packing, moving, and/or transferring fluids/materials. In some implementations, liquid-impregnated surfaces may be used to transfer fluids between containers. Liquid-impregnated surfaces may be used to convey viscous materials such as cement, oil, polymers, chocolate, cat food, dog food, waxes, and/or heavy oils in the oil and gas industry.

In some implementations, the no-slip condition between the conduit and the material being conveyed results in a velocity profile that is very steep at the edge of the conduit. For some processes, this is very undesirable since the high shear may actually degrade the material or product. The disclosed technology, in some implementations, provides a high-slip boundary condition so that the material moves, for example, as a slug, with a very uniform velocity profile. This is beneficial in applications with a very viscous material, and also with a low-viscosity material flowing in a small conduit, such as an IV drip.

In some embodiments, the liquid-impregnated surface coating may be applied to IV drips, the lining of IV tubes and the interior surfaces of IV bags. Such a coating would allow the content of an IV bag and/or tube to easily slide along the IV bag and tube with minimal waste of the content. By increasing the slipperiness of the IV lining, the liquid-impregnated surface coating reduces the attractive forces between the IV tubes and bags and their contents. This allows the content to be easily dispensed. For example, medical practitioners are frequently unable to convey an adequate flow rate of drugs to the patient because they cannot afford to put in a larger-gauge IV. Creation of a liquid-impregnated surface in the IV tube provides medical practitioners with the ability to convey an adequate flow rate of drugs to a patient without using a larger-gauge IV.

The applications of liquid-impregnated surfaces may, for example, be used for molding. For example, liquid-impregnated surfaces may be used in transfer molding. In some implementations, this includes a method for altering any existing surface to be a textured surface. In some implementations, a textured surface is filled with an uncured liquid polymer (for example). The filled surface (which is the mold) may be placed onto the existing surface so that the liquid polymer comes in contact with the existing surface. The liquid polymer may then be cured and hardened (which can be accomplished by a variety of processes dependent on the liquid polymer used). After hardening, the mold may be removed leaving textured cured polymer on the surface. In some implementations, the original textured surface (e.g., the mold) could be filled with a mixtures of nano or microscale particles and liquid (e.g., a slurry). Liquid-impregnated surfaces may also be used as a mold release agent.

The applications of liquid-impregnated surfaces may, for example, be used on bearings. For example, a thrust bearing could be comprised of two liquid encapsulated surfaces each with a different fluid that are immiscible. When the surfaces come in contact, the interface will be liquid-liquid. The resulting friction will be very low. A shear thinning fluid may be used to reach even lower frictions or to custom-tailor the resistance to vary.

The applications of liquid-impregnated surfaces may be used, for example, with high-friction manufacturing processes, containers, and associated equipment. For example, liquid-impregnated surfaces may be used for injection molding. Typically the high shear forces encountered in injection molding lead to viscous heating within the polymer melt, and in extreme cases may result in burning of the polymer. Application of a liquid-impregnated surface coating to mold runners and cavities will substantially reduce the shear forces at the wall, leading to lower injection pressures. This provides several benefits include reducing the energy requirements for injection, but it means that the same part may be molded on a smaller, less-expensive injection molding machine. In some implementations, the coating could simultaneously function as a mold release agent. In some implementations, power required to extrude from nozzles can be significantly reduced if the nozzle is coated with a liquid-impregnated surface. Liquid-impregnated surfaces may be used to create a lubricated die or extruder part that will not oxidize. Liquid-impregnated surfaces may also be used to lubricate the die.

Liquid-impregnated surfaces may be used for extrusion and/or forging. Extrusion and forging both typically involve the flow of a highly viscous material past a die surface, with a small amount of relative slip (as opposed to the large amount of relative slip in a conduit like a pipe). This includes extrusion and/or forging of plastics, metals, food products such as gum and candy, wire, glue, epoxy, rubber, and/or polymers. Liquid-impregnated surfaces may be used in cold extrusion and/or cold forging.

Liquid-impregnated surfaces may be used in container containing liquid metals, lubricants, caulk, cement, tiling grit, plaster, tar, asphalt, coal slurry, wax, laundry detergent, dishwashing detergent, grease containers, refrigerant containers, butane containers, liquid nitrogen container, liquid helium containers, gas containers, liquified gas container motor oil containers, petroleum product, brake fluid containers, oil containers, paint, inks, construction materials (e.g.,

intumescent), or other materials and/or containers. Liquid-impregnated surfaces may be used to create self-cleaning glass, photovoltaic cells, and/or solar thermal. Liquid-impregnated surfaces may also be used in association with desalination (e.g., condensers used in desalination), liquid natural gas condensers, energy conservation (e.g., condensers in power plants, oil pipelines, fuel lines. Liquid-impregnated surfaces may also be used to create de-icing surfaces on cars, airplanes, buses, and other vehicles and/or equipment. Liquid-impregnated surfaces may also be used for solid-to-solid friction reduction by applying creating a liquid-impregnated surface to one or both surfaces.

Liquid-impregnated surfaces may also be used in lab materials. For example, liquid-impregnated surfaces may be used in pipettes, beakers, and other materials. Liquid-impregnated surfaces may also be used in air filtration. For example, an air filter may use a replacement cartridge comprising liquid-impregnated surfaces (e.g., a weaving path of liquid-impregnated surfaces). The air filter cartridge may be incorporated into a device that moves air from the surrounding environment through the cartridge to filter the air. Dust passed through the air filter will, in some implementations, stick to the liquid-impregnated surfaces in the cartridge.

Liquid-impregnated surfaces for slug traps, insect traps, and/or pest traps. For example, liquid-impregnated surfaces may be used for capturing bugs. Encapsulated surfaces can produce a large normal force, insects landing on the surface will not be able to generate enough force to remove themselves. An encapsulated surface would have a similar effect and be extremely "sticky" for small insects. Liquid-impregnated surfaces may also be used as a pesticide by creating a surface that bugs cannot land on.

Liquid-impregnated surfaces may be used in adhesive strips. In some implementations, a liquid-impregnated surface has strong capillary adhesive forces in the normal direction (pulling the surfaces apart) resulting from the surface tension of the impregnating liquid pulling downward against the solid objects, normal to the surface. In some implementations, the textured surface may be encapsulated with liquid that can solidify or cure(for example an epoxy). Thus a curable liquid encapsulated surface could be as convenient to apply as a conventional tape, but have the strength of epoxy.

Liquid-impregnated surfaces may be used for display preservation. For example, a user can write/print/draw on a properly textured surface. After the surface is coated in an oil, the writing is effectively locked in and protected.

Liquid-impregnated surfaces may also be used on windshields of planes, trains, or automobiles, or similarly on helmet visors and/or glasses (e.g., sunglasses). For example, cleaning windshields in rain with wipers requires significant amount of energy. Liquid-encapsulated windshields can potentially repel rain drops without the need for wipers. Similarly, liquid-impregnated surfaces may be applied to airplane windshields as well. A liquid-impregnated surface may be applied to the windshield or may be sprayed from a reservoir onto the windshield when necessary (e.g., during an airplane landing, during a rainstorm, etc.).

Liquid-impregnated surfaces may also be used on skis, ice skates, sleds, swimsuits, boats, or other water and/or sporting equipment. Liquid-impregnated surfaces could also be used on the surface of torpedos.

Liquid-impregnated surfaces may also be used in tractors for farming, $CO_2$ condensers, LP condenser, N2 condensers, surfaces for condensation of gases and/or on the interior of hydraulic lines and gas lines.

Liquid-impregnated surfaces may be used on household appliances, accessories, and items such as pans, cookware, eating utensils, spatulas, pots, plates, drains, and/or toilet bowls. Liquid-impregnated surfaces may be applied to a portion of a surface to corral liquids. Liquid impregnated surfaces may be used on toys and games. For example, liquid-impregnated surfaces may be used on a slip n slide.

In some embodiments, the liquid-impregnated surface is created by applying a uniform layer of the impregnating liquid to any surface. This surface may be the surface of a contact lens. Liquid encapsulated surfaces could be applied to a contact lens to improve the comfort on the wearer. Liquid encapsulated surfaces would also help contact lenses retain moisture and maintain a tear film within the eye to prevent dry eye symptoms including burning, stinging, redness, foreign body sensation, excess tearing, and intermittent blurred vision, and reduce potential scratching of the eye.

Currently, the lifetime of disposable contact lenses is two weeks on average. Liquid encapsulated surfaces may extend the lifetime of current disposable contact lenses substantially. The retained liquid interface between the contact lens and the eye would help reduce contact lens wear and tear, thereby improving the contact lens's lifetime. The liquid encapsulated surfaces may allow the contact lenses to be worn overnight and for periods of longer than two weeks.

In some embodiments, liquid encapsulated surfaces may also reduce contact lens maintenance. Currently rewetting drop products such as "Refresh Contacts", "Clerz Plus", or "Clear Eyes Contact Lens Relief' moistens contact lenses and removes particles accrued on the contact lens that cause irritation and discomfort. However, these rewetting drops will not be needed as frequently with liquid encapsulated surfaced contact lenses since the liquid encapsulated surfaces will retain moisture and prevent dry eyes. Current contact lenses require soaking in a saline solution nightly to moisturize the contact lens. Such a nightly soaking may not be necessary due to the liquid encapsulated surface present in the improved contact lenses.

In some embodiments, the contact lenses may have texture or roughness on one or both sides of the lens, or porosity extending all the way through the lens. The liquid to be housed in the liquid layer of the lens could be applied to one or both sides of the lens. Alternatively, the liquid could be soaked all the way through the lens. The liquid may be applied and reapplied by the user after purchase multiple times.

In some embodiments, the contact lens is constructed from polyimide. The texture can be controlled or adjusted via a temperature- or solvent-induced crystallization of the polymer surface of polyimide to form spherulites or other fine microstructures. Many polymers already used in the manufacture of contact lenses undergo spherulitic crystallization.

The solid and liquid materials may be chosen from materials already deemed safe by the United States Food and Drug Administration for contact with the eye. The liquid could be immiscible with eye fluid and the eye fluid may act as the supply to the textures.

In some embodiments, the solid features and the material of the lens itself may be polymer, hydrogel, polyimide, polymacon, silicone hydrogel, polymethyl methacrylate (PMMA or Perspex/Plexiglas) or any combination of these materials.

In some embodiments, optical clarity could be achieved either by having features smaller than 100 nm or by matching the refractive index of the texture material and the liquid. The liquid and texture would ideally be transparent, or translucent, but thin enough so that the effective transmissivity within the visible spectrum is at least 95%.

In some embodiments, the impregnating liquid in the liquid layer is colored. The colored impregnating provides the color for colored contact lenses.

In some embodiments, the impregnating liquid forms a liquid layer extending above the top of the solid features of the surface while at equilibrium or substantially at equilibrium. In some embodiments, the liquid layer extends above the top of the solid features by at least about 5 nm.

In some embodiments, current laser etching techniques, such as $CO_2$ or Deep UV, can be adapted to generate patterned and textured surfaces across the entire interior surface of the contact lens. Current laser etching techniques only create small identification marks on the inside of a contact lens. The laser techniques may be expanded to provide a patterned textured with uniform dimensions across the entire contact lens. Impregnating this textured surface with a liquid with the same or almost the same refractive index as the contact lens material would cause the contact lens to become transparent. An example experiment discussed below compares the transparency of a contact lens with a liquid encapsulated surface to that of a conventional uncoated contact lens.

In certain embodiments, the particles are coated onto the medical device or medical implement's surface by spray coating the surface with an impregnating liquid solution. The spray coating may apply a uniform coat of impregnating liquid to the surface of the medical device or medical implement. In certain implementations, the impregnating liquid may be spray coated onto the surface of the medical device in multiple stages. In certain implementations where the impregnating solution is composed of several different solutions, the various constituent solutions of the impregnating liquid may be spray coated onto the target surface in different stages.

The applications of liquid-impregnated surfaces for inhibiting nucleation may include, for example, preventing of nucleation of plaque on teeth, dentures, braces, or retainers. The applications of liquid-impregnated surfaces my also include, for example, preventing fibrosis on artificial implants. Furthermore, applications may also include preventing thrombosis on surfaces in contact with blood, or surfaces of tubes or artificial arteries or stents, which clog from build up of cholesterol or other solid-like materials. These surfaces would benefit from a more lubricated interface.

In some embodiments, the liquid-impregnated surface is created by applying a uniform layer of the impregnating liquid to a surface. In certain implementations, this surface may be human or animal tissue. A uniform layer of impregnating liquid may be sprayed onto a surface to create a uniform liquid-impregnated surface coating.

In some embodiments, the liquid-impregnated surface coating may be applied to the internal surface of a syringe for emptying out the contents of the syringe. For example, the internal surface of the syringe cylinder's barrel may be coated with the impregnating liquid. This will reduce the attractive forces between contents of the syringe and internal surface of the syringe cylinder's barrel to expel the maximum amount of the syringe's contents with less applied plunger force.

In some embodiments, the liquid-impregnated surface coating may be applied to an artificial or natural lining for artery walls to prevent plaque formation. The lining may be coated with the impregnating liquid in such a manner that does not allow plaque to stick to the lining of the artery walls easily. The impregnating liquid may be applied to the lining of the artery walls by pumping the artery walls with the impregnating liquid solution or by any surgical procedure.

In some embodiments, the liquid-impregnated surface coating may be applied to IV drips, the lining of IV tubes and the interior surfaces of IV bags. Such a coating would allow the content of an IV bag and/or tube to easily slide along the IV bag and tube with minimal waste of the content. By increasing the slipperiness of the IV lining, the liquid-impregnated surface coating reduces the attractive forces between the IV tubes and bags and their contents. This allows the content to be easily dispensed. For example, medical practitioners are frequently unable to convey an adequate flow rate of drugs to the patient because they cannot afford to put in a larger-gauge IV. Creation of a liquid-impregnated surface in the IV tube provides medical practitioners with the ability to convey an adequate flow rate of drugs to a patient without using a larger-gauge IV.

In some embodiments, the liquid-impregnated surface coating may be applied to colostomy bags. This allows for smooth reception of fecal discharge after colostomy more easily and reduces discomfort to the patient.

In some embodiments, the liquid-impregnated surface coating may be applied to teeth to prevent buildup of plaque. By applying such a coating, food particles and other plaque will be less likely to stick to teeth, thereby increasing dental health of the subject.

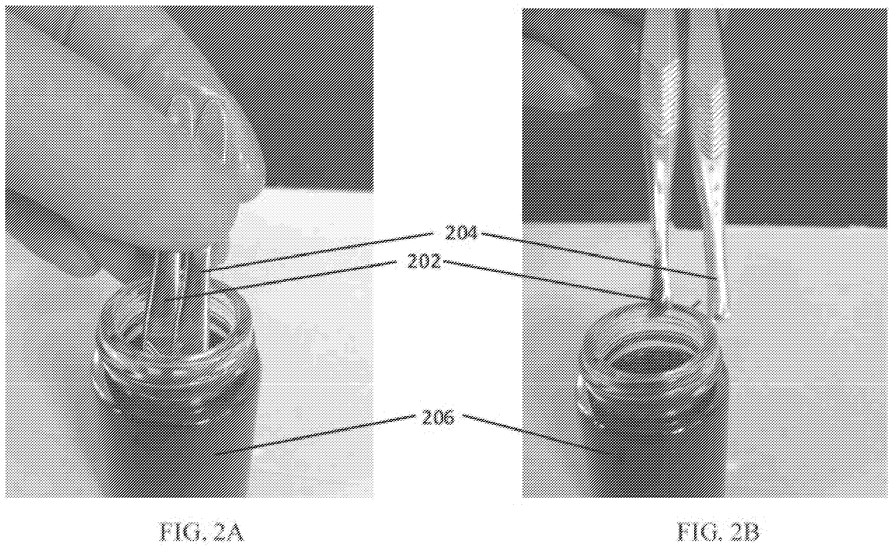

In some embodiments, the liquid-impregnated surface coating may be applied to metal or metallic surgical instruments, as shown by the experiment documented in FIGS. 2A and 2B as discussed below. Applying such a coating to surgical instrument allows bodily fluids such as blood to be repelled off the coated surgical instruments and allows the instrument to remain clean.

In some embodiments, the liquid-impregnated surface coating may be applied to bandages in order to allow the bandages to be easily removed from the skin without causing discomfort to the patient. For instance, bandages with such a liquid-impregnated surface coating do not become glued to the wound or skin very tightly over time and pressure and can be easily removed.

In some embodiments, the liquid-impregnated surface coating may be applied to blood pumps. Shear forces encountered in pumping blood and other biological fluids often damage or destroy cells by mechanically ripping them apart. The liquid-impregnated surface coating significantly reduced the shear forces at the surfaces of the pump to prevent damage of cells and other biological structures.

In some embodiments, the liquid-impregnated surface coating may be applied to lab supplies and pharmaceuticals in order for them to remain clean and prevent foreign substances from sticking to them.

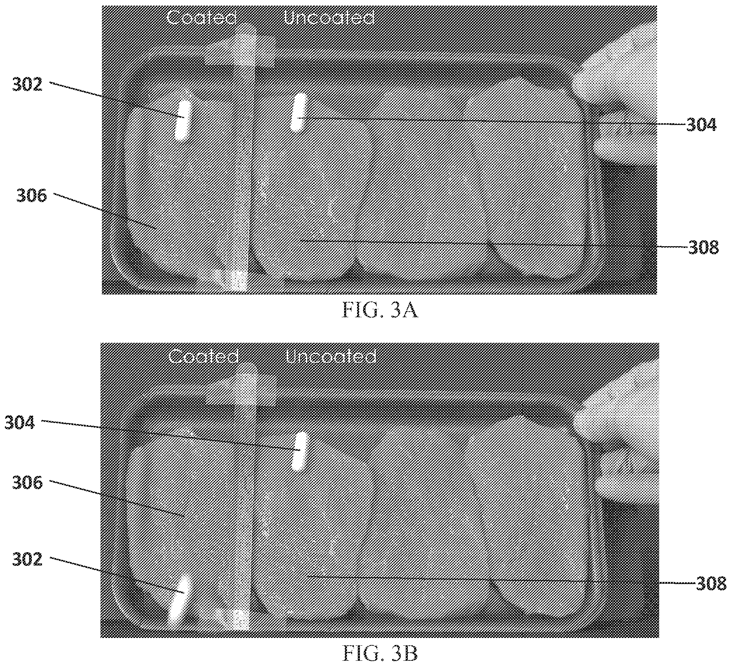

In some embodiments, the liquid-impregnated surface coating may be applied to pills and capsules for ease of swallowing , as shown by the experiment documented in FIGS. 3A and 3B as discussed below. Applying such a coating to pills allows the coated pills to easily slide along the tongue and esophagus tissue by reducing the frictional force between the pill and human tissue.

In some embodiments, the liquid-impregnated surface coating may be applied to micropipettes, pipettes, pipette tips, small volume containers of biological fluids and samples. For small-volume containers and micropipettes, the proportion of the contents that remains stuck to the container represents a significant fraction of the total volume of the container. Furthermore, the contents are often expensive and labor-intensive to obtain. Applying the impregnated liquid coating to the interior surfaces of these containers and pipette tips allows the contents to be easily expelled from these containers with minimal waste. Similarly, the coating may also be applied to the contents of these containers, especially DNA and RNA strands which will aid in the easy expulsion of these compounds from the small volume containers.

In some embodiments, the liquid-impregnated surface coating may be applied to microfluidic devices. Often microfluidic channels become clogged with the contents being passed through these channels. By coating the microfluidic channels with liquid-impregnated surface coating, the contents of these microfluidic channels do not clog the channels and the microfluidic devices can remain operation for a longer period of time without any maintenance.

In some embodiments, the liquid-impregnated surface coating may be applied to dialysis tubes and other components of dialysis machines to facilitate easier for waste and excess water removal from the blood.

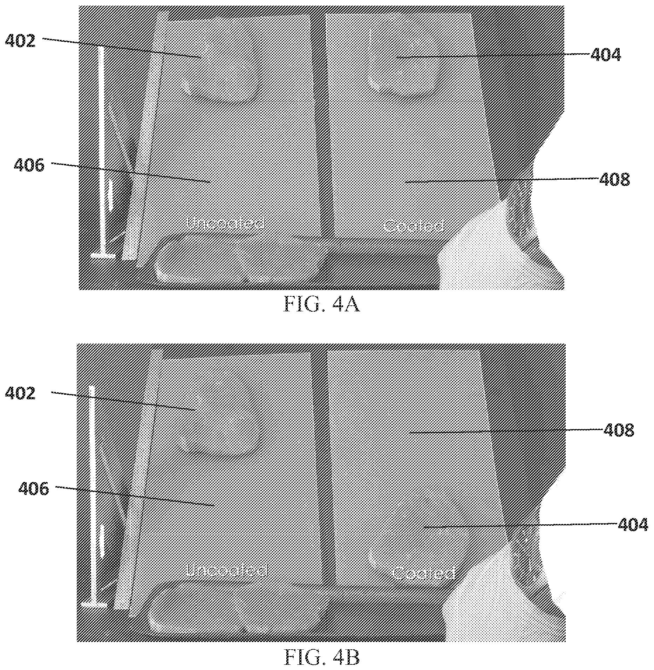

In some embodiments, the liquid-impregnated surface coating may be applied to any surgical tools that are inserted into the body such as endoscopes, stents, syringe needles, stents, catheters, tracheotomy tubes, and intubation devices. Such a coating, when applied to these surfaces, allows for a much easier insertion into the body without causing any undesired tears in body tissue. Such a coating allows for more comfortable insertion of intubation equipment. The encapsulated liquid in the coating may also contain mid antiseptic and an anesthetic that would allow for the local area of insertion to be anesthetized and clean while the surgical tool is being inserted. An experimented conducted using polypropylene sheets, used to simulate the surface of a surgical instrument, in contact with a steak, used to simulate human tissue, is shown in FIGS. 4A and 4B as discussed below.

In some embodiments, the liquid-impregnated surface coating may be applied to creams, prescription creams, ointments, Neosporin, triple antibiotic ointment, burn relieving cream, anti-itch cream, aloe-vera gel, sunscreen lotion, and other lotions. The coating may also be applied to containers of ointments, lotions, and creams. Such a coating would allow these chemicals to be easily dispensed and would prevent the last few drops of such cream, lotion of ointment to stick to the container walls.

In some embodiments, the liquid-impregnated surface coating may be applied to medical supplies, gloves, bandages for covering open wounds, bandages for skin conditions, medical implants, implant coatings in order to keep them clean from foreign particles.

In some embodiments, the liquid-impregnated surface coating may be applied to medical device surfaces, artificial heart, and artificial organs to prevent buildup of organic matter on these devices.

In some embodiments, the liquid-impregnated surface coating may be applied to prosthetics and self-lubricating joints in order to keep them free of dirt and organic matter buildup that could deteriorate effective operation.

In some embodiments, the liquid-impregnated surface coating may be applied to orthodontic tools such as a retainer, a tooth mold, dentures, dental braces, invisible braces. The surface coating can avoid plaque buildup on the surfaces of these orthodontic tools improving dental health and hygiene.

In some embodiments, the liquid-impregnated surface coating may be applied to bridges and wetted surfaces to avoid biofouling.

In some embodiments, the encapsulated liquid in the liquid-impregnated surface coating may be antiseptic and antibacterial in order to allow the surfaces to remain clean. This is particularly important in medical applications where the cleanliness of medical devices is paramount.

In some embodiments, the liquid-impregnated surface coating may be applied to adhesive strips. Liquid impregnated surfaces have strong capillary adhesive forces in the normal direction. The lateral forces depend on the impregnated liquid viscosity. An extremely high viscosity impregnated liquid can behave essentially as a solid, preventing sliding, and therefore the surface would behave similarly to tape. Low viscosity fluids slide easily, thus resulting in an surface that behave as an adhesive in the normal direction but slides laterally . Alternatively, the textured surface could be encapsulated with liquid that can solidify or cure(i.e., as an epoxy). Thus a curable liquid encapsulated surface could be as convenient to apply as a conventional tape, but have the strength of epoxy.

In some embodiments, the liquid-impregnated surface coating may be applied to condoms. The protective coating could allow for reduced friction during intercourse and could prevent tearing. Additionally, the surface coating could be applied to similar adult paraphernalia that is inserted into bodily orifices to reduce friction and minimize tearing.

In some embodiments, the liquid-impregnated surface coating may be applied to drug release patches. This product might appear similar to a Band-aid, but the white portion of the Band-aid would be replaced with a liquid encapsulated surface. The encapsulated liquid can be medication or drug. It can then be applied to the skin to deliver the medication.

In some embodiments, the liquid-impregnated surface coating may be applied to cosmetic products such as nail polish, shampoo, conditioner, body wash, hair gel, facemasks, and toothpaste. Applying such a coating to these cosmetic products allows them to repel dust and prevents dust that would otherwise be attracted to them to be applied to the body.

One may control the mechanical interactions between the surface of a medical device or implement and a tissue or fluid in contact with said surface. The fluid may be biological in origin (including but not limited to blood, saliva, sweat, urine or interstitial fluids), or it may be a man-made suspension or solution of bioactive components (including but not limited to drugs, vitamins, minerals, proteins, peptides, or nucleic acids) in a fluid.

In one embodiment, the lubricity of the surface of a medical device or medical implement is modified in such a way that the lubricity to a contacting tissue or fluid is either increased or decreased, relative to the unmodified surface. In this way the effective coefficient of friction of the surface to a tissue or fluid can be increased or decreased in a controllable way. For example, if it is desired that a surface of a device or implement move easily across the surface of a tissue or fluid then its effective coefficient of friction can be reduced; in a similar way if it is desired that a surface of device or implement not move easily across the surface of a tissue or fluid (e.g., effectively anchoring the device or implement to the tissue or fluid) then its effective coefficient of friction can be increased.

In one embodiment, a medical device or medical implement is provided with controlled lubricity to tissue, or biological fluid, or fluid with suspended or dissolved bioactive components, including drugs, the device or implement comprising a surface comprising an impregnating liquid and a plurality of micro-scale and/or nano-scale solid features spaced sufficiently close to stably contain the impregnating liquid therebetween, wherein said impregnating liquid fills spaces between said solid features, wherein said surface stably contains said impregnating liquid between said solid features, and wherein said impregnating liquid is substantially held in place between said plurality of solid features regardless of orientation of said surface.

In another embodiment of the invention, the lubricity of the device or implement is varied in spatial dimensions for the purposes of spatial control over the movement of tissues or fluids across the surface of the device or implement. In one example, it may be desired that fluids flow easily over some portions of the surface of the device or implement while being substantially pinned or trapped on other portions of the surface of the device or implement. In another, it may desired to control the interactions between the surface of a device or implement and a tissue such that the tissue moves freely over some regions of the surface while it remain substantially anchored to other regions of the surface.

In one embodiment, the properties of the surface and/or of the impregnating liquid are varied in spatial dimensions such that patterns of different lubricity are obtained.

In another embodiment of the invention the surface of a tissue itself is modified to control the lubricity between the tissue and the surface of a medical device or implement, or to the motion of a fluid upon the surface of the tissue. The surface of the tissue can be modified using mechanical, chemical, electrical or other forces to introduce into the surface of the tissue a plurality of micro-scale and/or nano-scale solid features. These features are sufficient to effectively and stably retain an impregnating liquid, the properties of which allow control over the lubricity between the tissue modified in this way and the surface of a medical device or implement, or to the motion of a fluid upon the surface of the tissue. This modification of the tissue surface may result in either a temporary or permanent change in the lubricious properties of the tissue to a surface of a device or implement, or to a fluid.

In one embodiment, a tissue is provided with controlled lubricity to medical devices or medical implements, or biological fluids, or fluids with suspended or dissolved bioactive components, including drugs, the tissue comprising a surface comprising an impregnating liquid and a plurality of micro-scale and/or nano-scale solid features spaced sufficiently close to stably contain the impregnating liquid therebetween, wherein said impregnating liquid fills spaces between said solid features, wherein said surface stably contains said impregnating liquid between said solid features, and wherein said impregnating liquid is substantially held in place between said plurality of solid features regardless of orientation of said surface.

In one embodiment, the properties of the surface and/or of the impregnating liquid are varied in spatial dimensions such that patterns of different lubricity are obtained.

Experimental Examples

*Example 1*

FIG. 2 shows experimental measurements of blood droplet repulsion from tweezers coated with the liquid-impregnated surface coating. As shown in FIG. 2A, two identical plastic tweezers, tweezer 202 and tweezer 204 are dipped into container 206 which is filled with two drams of pig blood. Tweezer 202 is uncoated as a control tweezer. Tweezer 204 is coated with the liquid-impregnated surface coating. Tweezers 202 and 204 are dipped into container 206 at the same time and removed at the same time. Tweezers 202 and 204 are held in the same hand.

FIG. 2B shows the effect of the surface coating on tweezer 204 when both tweezers 202 and 204 are removed from container 206 of pig blood. The uncoated tweezer 202 is stained with blood residue. The liquid-impregnated surface coated tweezer 204 shed the majority of the blood away with minimal reside as soon as tweezer 204 was withdrawn from container 204.

The experiment of FIG. 2 demonstrates that liquid-impregnated surfaces can be engineered to keep medical devices clean of bodily fluids. This is helpful in keeping medical equipment and surgical tools sterile.

*Example 2*

This example demonstrates liquid-impregnated surface pills are easier to swallow than uncoated pills. It demonstrates this by comparing the sliding speed of a liquid-impregnated surface coated pill on a piece of steak against the sliding speed of an uncoated pill.

FIG. 3A shows a screenshot of a video taken to document coated pill 302 and uncoated pill 304 sliding on steak to mimic the esophagus and tongue tissue. The coated and uncoated pills were placed in a parallel orientation on two pieces of steak, steak 306 and 308 as show in FIG. 3A. Steak 306 and 308 were placed on an incline of 65°. Pill 302 was coated with a liquid impregnated surface (carnauba wax and ethyl oleate) whereas pill 304 was uncoated as a control.

In particular, tweezers were used to pick up the cylindrical pale yellow pills (Vitacost Alpha Lipoic Acid & Acetyl L-Carnitine HCl -- 1600 mg per serving). Carnauba wax was sprayed onto pill 302 for three seconds to apply uniform coating of the liquid-impregnated coating. Nitrogen gas was blown across pill 302 to allow time to dry coating prior to application of ethyl oleate. Ethyl oleate was sprayed onto pill 302 for three seconds to apply uniform coating. Subsquently, uncoated pill 304 was placed onto steak 308. Liquid-impregnated surface coated pill 302 was placed onto steak 306. Pills 302 and 304 were placed at top of their respective steak. The orientation of the pills was perpendicular to ruler 310. Subsequently, steaks 306 and 308 were adjusted to a 65 degree inclined plane. FIG. 3A shows pills 302 and 304 at zero seconds as soon as they were placed on the top of the steaks.

FIG. 3B shows a screenshot 3.5 seconds after pills 302 and 304 were placed on the top of the steaks. At this time, coated pill 302 reached the bottom of steak 306 while uncoated pill 304 remained at the top of steak 308. Pill 302 started sliding slowly but rapidly accelerated to a rate of ~4.5 cm/s (calculated based on a travel distance of 7 cm over 1.5 seconds. The uncoated pill remained at the top of the steak throughout the experiment.

The experiment of FIG. 3 demonstrates that liquid-impregnated surface coatings on pills helps the pill slide on top of animal tissue such as steak which mimics the surface structure of the human tongue and esophagus since the uncoated pill did not travel any distance on the steak at the same inclined angle.

*Example 3*

This example demonstrates the low friction between liquid-impregnated surfaces and flesh. This is demonstrated by comparing the sliding speed of raw eye round steak on a liquid impregnated surface with the sliding speed of another raw eye round steak on an uncoated surface. A video was taken was to document the steaks' motion on uncoated and coated polypropylene sheets.

This experiment was performed by first cutting a 12" x 12" polypropylene (PP) sheet (Gauge = 0.060") into two 6" x 12" sheets, sheet 406 and 408. Carnauba wax was sprayed onto sheet 408 for fifteen to thirty second to apply uniform coating. Subsequently, ethyl oleate was sprayed onto sheet 408 for thirty to forty five second to apply uniform coating. Sheet 406 was left uncoated as control. Sheet 406 was placed next to sheet 408 and both sheets 406 and 408 were placed on a 45 degree incline. Steak 402 was placed on top of sheet 406 and steak 404 was placed on top of sheet 408. The beginning of the meat steaks were four inches from the top of the PP sheets. Video of the meat travelling was taken to document the difference between uncoated and coated PP sheets. FIG. 4A shows a video frame at time zero when the steaks were placed on top of the PP sheets. FIG. 4B shows a video frame one hundred and thirty one seconds after the frame shown in FIG. 4B. FIG. 4B shows that steak 404 has reached the bottom of sheet 408 while uncoated steak 402 still remains near the top of the PP sheet 406.

Time was measured for steak to travel eight inches to the bottom of the PP sheet. Steak 404 on PP sheet 408 took 131 seconds to travel to bottom of sheet. The average velocity of liquid-impregnated surface coated steak 404 on sheet 408 was 0.055 inches/sec. Uncoated steak 402 on PP sheet 406 slightly moved but remained about seven inches from the bottom of the sheet after 2 minutes and 30 seconds. Upon additional time (~5 mins), the steak did not appear to move any further down the inclined ramp.

The experiment of FIG. 4 demonstrates that liquid-impregnated surface coatings on surfaces helps animal flesh slide down the surface more easily than on uncoated surfaces. This provides evidence to prove that such a liquid-impregnated surface coating provides reduction of shear forces to prevent damage to cells and other biological structures in blood or other biological fluids being pumped.

### Example 4

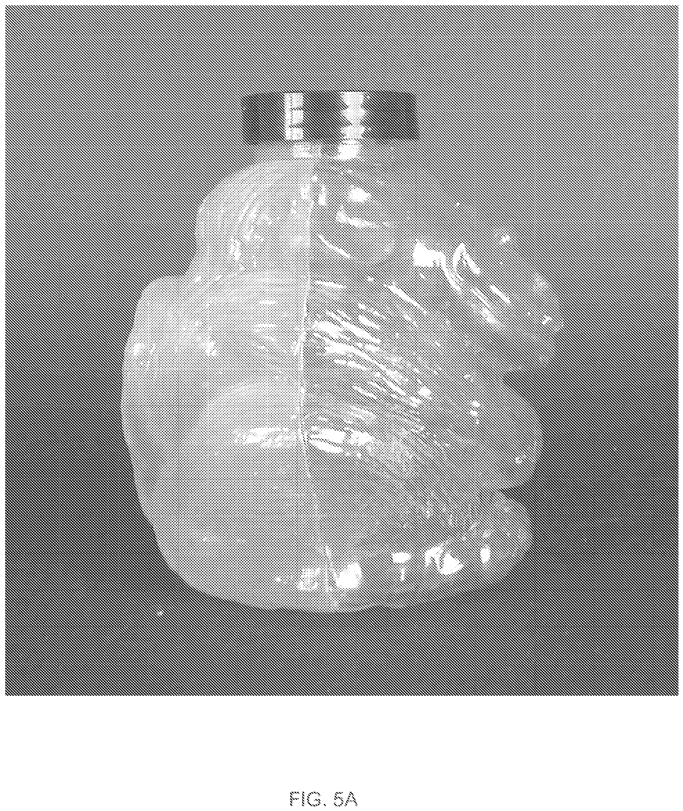

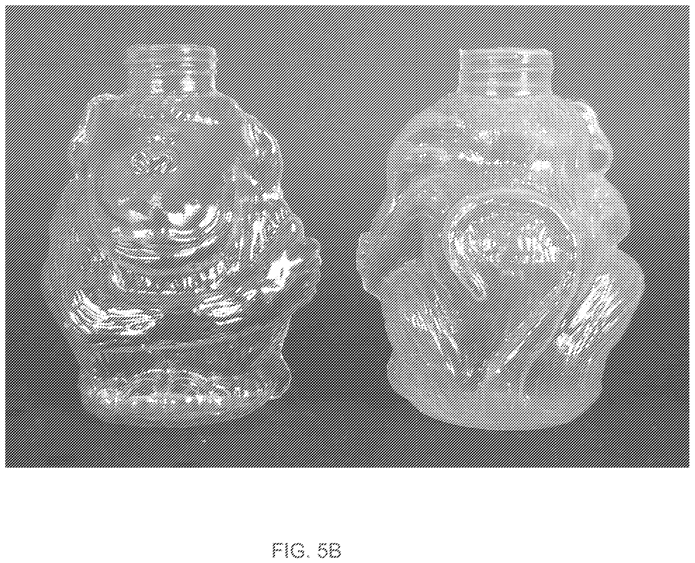

FIGS. 5A-D illustrate a mold-release experiment using concrete and a liquid-impregnated surface coated mold. In some embodiments, the liquid-impregnated surface coating may be applied to orthodontic tools such as a tooth mold. A heavily detailed plastic bottle in the shape of a monkey, complete with crevices and structures, was used to demonstrate liquid-impregnated surfaces as mold release/non-stick coatings as shown in FIG. 5A. The approximately 500ml HDPE, monkey-shaped bottle was sawed in half with a reciprocating saw to create a front half and a back half as shown in FIG. 5B. The back half of the bottle was coated with a liquid impregnated surface, described below, while the front half remained uncoated.

A liquid impregnated solution was sprayed onto the back half of the bottle. The liquid-impregnated solution was prepared by using adding 1.5 g of fluorinated wax (HF diblock grey, Toko) to 80 ml of toluene and heated on a hot plate until total dissolving of the wax. Next, the solution was sonicated for 5 minutes and was let to cool down to room temperature. Finally 10 g of PTFE particles (1 $\mu$m size, Sigma) were added and sonicated for 5 minutes more. The solution was sprayed onto the mold to create a coating of approximately 10 um thickness, and then Galden HT 200 was sprayed to impregnate and fill the textures.

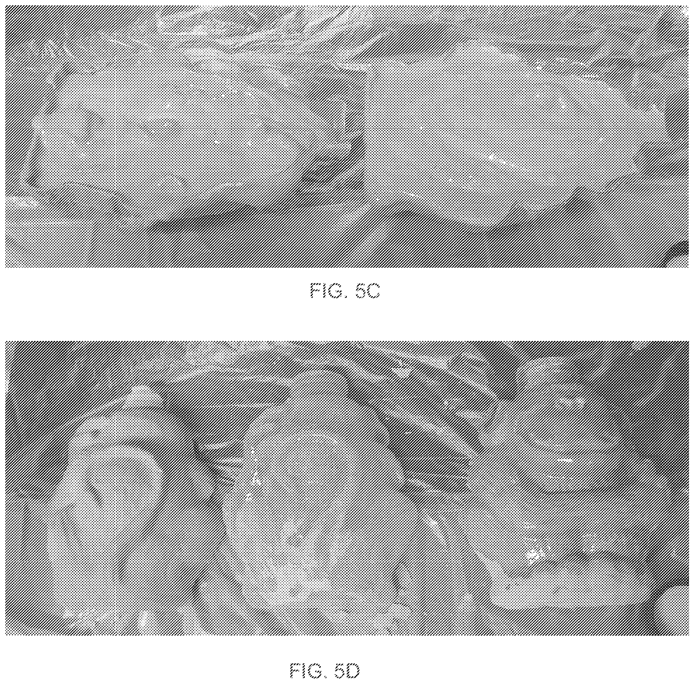

Rapid setting concrete was mixed per the manufacturer's instructions and poured into each mold until full as shown in FIG. 5C. The concrete was left to cure for approximately 15 minutes at room temperature (70°F) and each mold was turned upside-down on the counter. We then pulled the coated plastic mold from the hardened concrete easily and completely, leaving behind a cast of the inside of the bottle as shown in FIG. 5D. The uncoated side would not release from the mold.

### Example 5

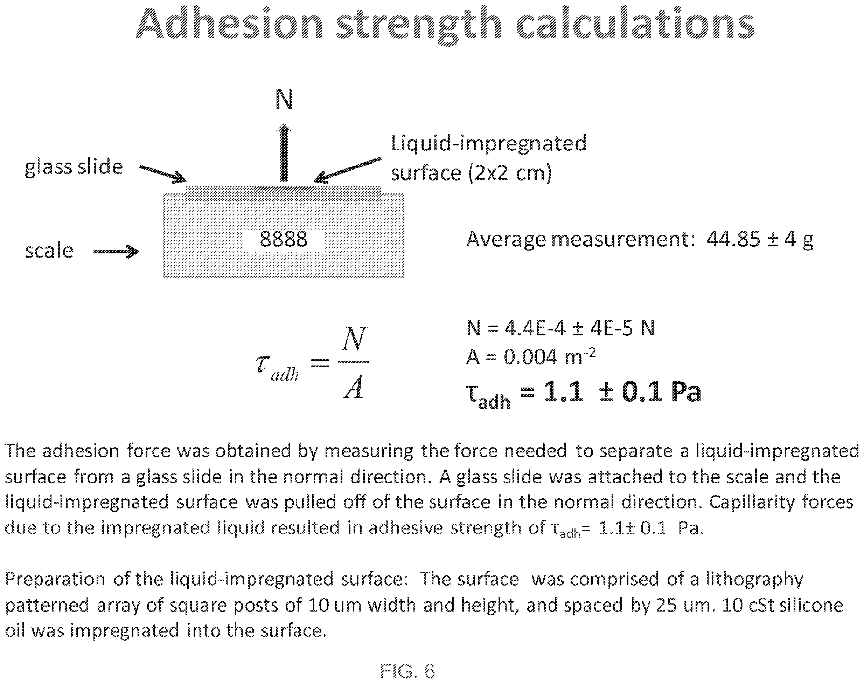

FIG. 6 illustrates a solid-to-solid adhesion experiment. The lateral forces (sliding) depend on the impregnated liquid viscosity. An extremely high viscosity impregnated liquid can behave essentially as a solid, preventing sliding, and there for the surface would behave similarly to tape (FIG. 6). Low viscosity fluids slide easily, thus resulting in a surface that behave as an adhesive in the normal direction but slides laterally (Imagine an air hockey table where the mallets can easily slide but are extremely difficult to pull off).

The adhesion force was obtained by measuring the force needed to separate a liquid-impregnated surface from a glass slide in the normal direction. A glass slide was attached to the scale and the liquid-impregnated surface was pulled off of the surface in the normal direction. Capillarity forces due to the impregnated liquid resulted in adhesive strength of $\tau_{adh}= 1.1 \pm 0.1$ Pa. The liquid-impregnated surface was prepared using a lithography patterned array of square posts of 10 um width and height, and spaced by 25 um. 10 cSt silicone oil was impregnated into the surface.

We measured the static coefficient of friction, $\mu_s$, between two solid materials with three different configurations. The first interface is silicon on PET (configuration 1), the second interface is silicon with the liquid impregnated surface (for which the normal adhesion was measured) on PET, and the third interface (configuration 3) is glass sprayed with carnauba wax to create a textured surface that was impregnated with ethyl oleate. The PET surface beneath was coated with a thin film of toothpaste to yield a chemistry that is preferentially contacted by ethyl oleate over the carnauba wax, insuring a stable liquid film between the solid materials. The coefficient of friction for each of these configuration was calculated as $\mu_s = \tan\alpha_{slide}$, where $\alpha_{slide}$ is the angle at which the surface first begins to slide. A weight was attached to the top of each surface resulting in a force per unit area of the top surface of around $520\pm10$ N/m$^2$ on each surface.

The slide-off angles, $\alpha_{slide}$, for configuration 1, 2, and 3, were 24°, 16°, and 7° respectively resulting in coefficients of friction, $\mu_s = \tan\alpha_{slide}$ of 0.44, 0.29, and 0.12 respectively. Thus configurations 2 and 3 both produced lower coefficients of friction than the direct solid/solid interface (configuration 1) Configuration 3, for which the chemistry of the bottom was modified with a layer of toothpaste, had the lowest friction - presumably because the a thin film of liquid (ethyl oleate) is stable between toothpaste and the carnauba wax, and therefore there was no solid-to-solid contact.

*Example 6*

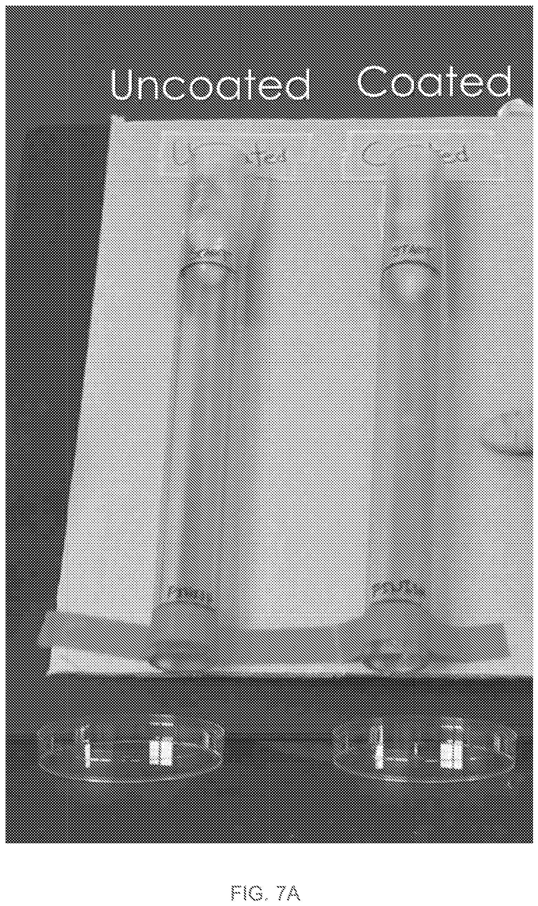

FIGS. 7A-F demonstrate the effect of liquid-impregnated surfaces on tubes, pipes, channels, or other similar items. A PVC pipe was cut into one foot sections. A carnauba wax coating (carnauba wax suspended in trichloroethylene, 5% wt.) was sprayed into both ends of the pipe for 5-20 seconds to apply a uniform coating to the inside of the pipe. N2 was blown across and within the pipe to allow coating to dry (~1-2 minutes). Next, ethyl oleate was sprayed into both ends of the pipe for 5-20 seconds until the pipe becomes transparent again (which indicates the texture is fully impregnated). The resulting product was a pipe with a liquid-impregnated inner surface.

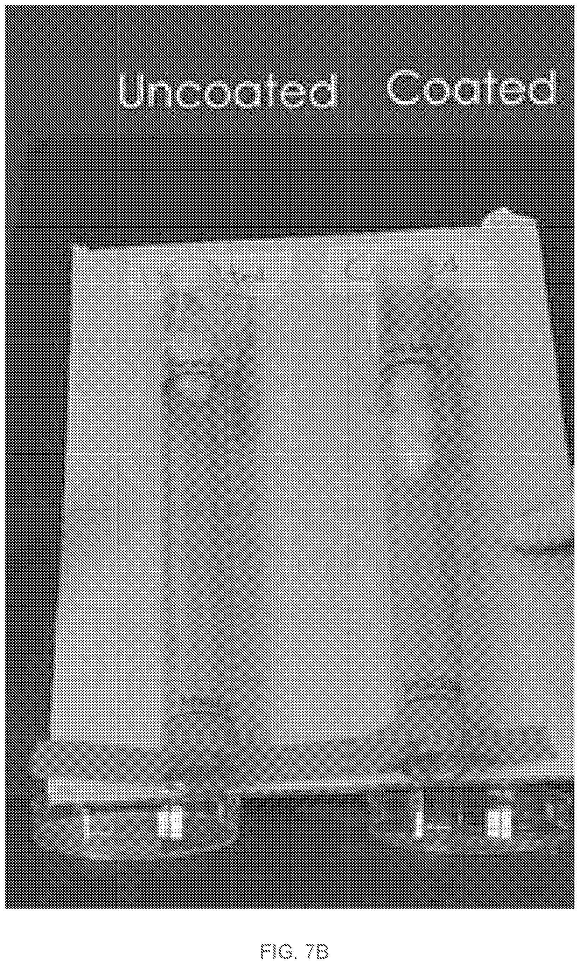

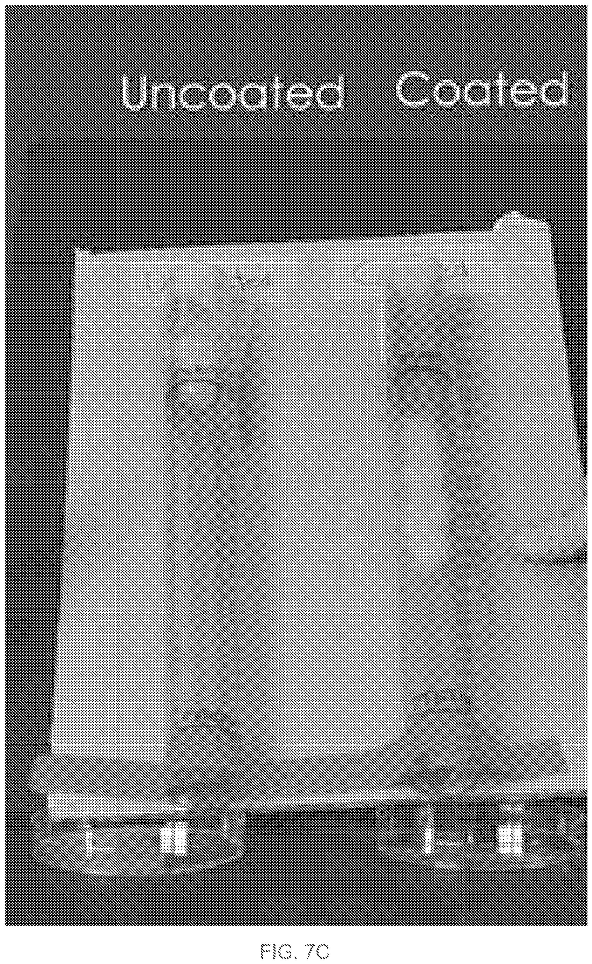

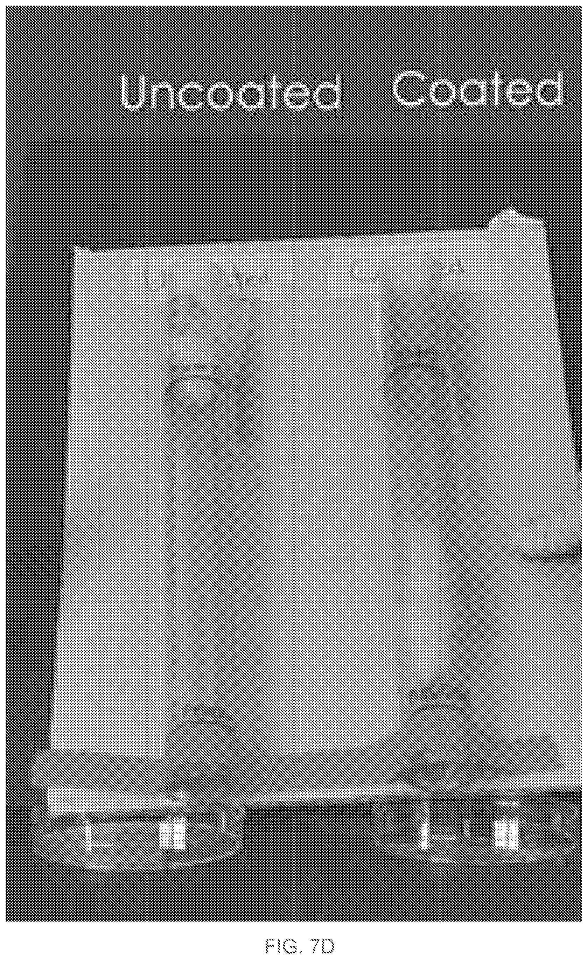

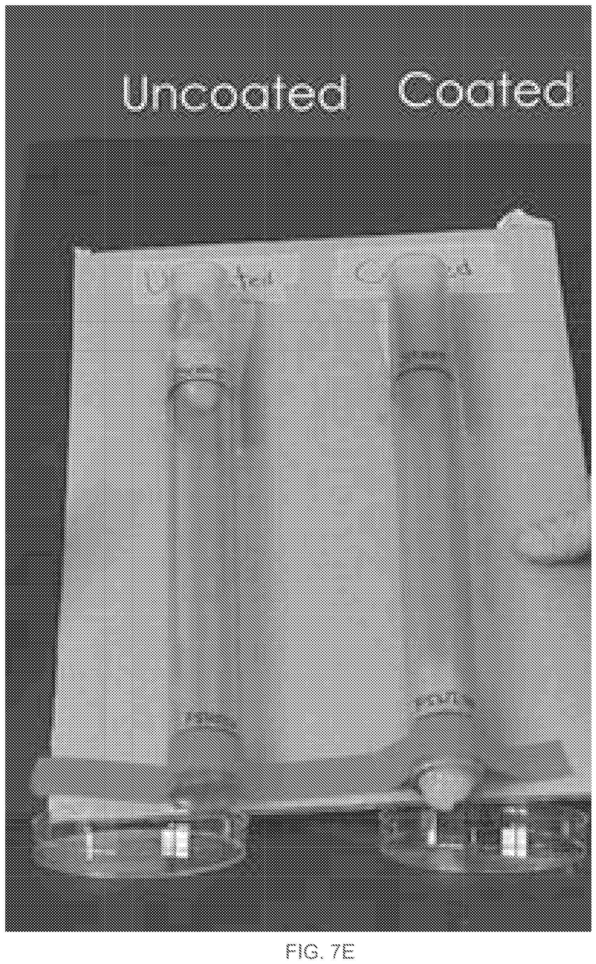

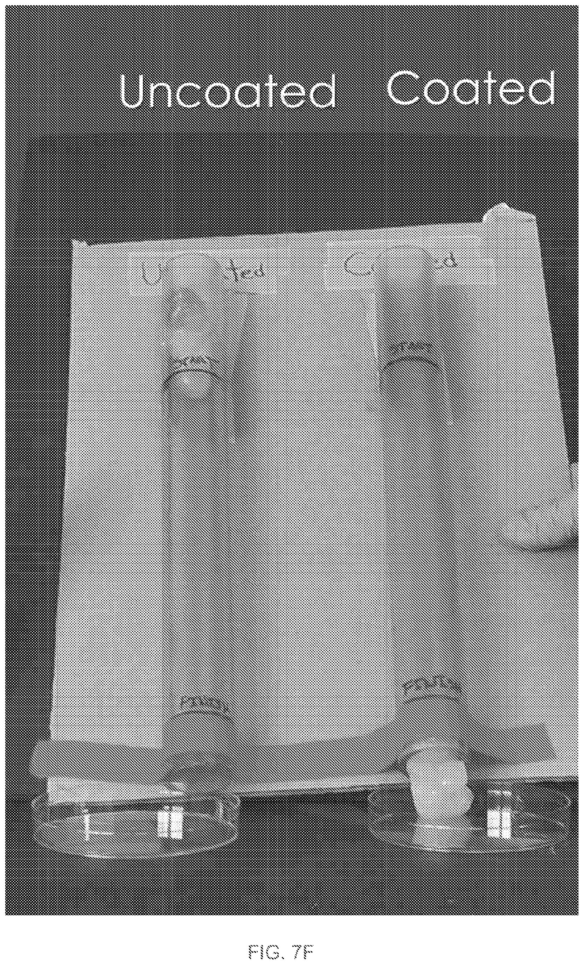

The liquid-impregnated pipe and a unmodified pipe of equal length were assembled in a pipe testing apparatus. The unmodified pipe was used as a control to assess the impact of the liquid-impregnated pipe. The two pipes were fixed to a cardboard backing use tape was shown in FIG. 7A. Labels "Start" and "Finish" were applied at the same positions on both pipes. The distance separating the labels on each pipe was 20 cm. Twenty grams of toothpaste was added at the start line of both tubes. The pipe testing apparatus was placed at a 45 degree angle and the velocity of the toothpaste as well as the time it took the toothpaste to travel 20 cm from the start to finish lines was measured. FIGS. 7B-E illustrate the position of the toothpaste in the uncoated pipe and the coated pipe over time. FIG. 7B illustrates a total elapse time of .4 seconds. FIG. 7C illustrates an elapse time of .9 seconds during which the toothpaste in the coated pipe slide approximately 10 cm while the toothpaste in the uncoated pipe remained at the start line. FIG. 7D illustrates the toothpaste in the coated pipe near the finish line after 1.9 seconds from starting the experiment. After a total elapse time of 2.1 seconds, the toothpaste in the coated pipe is at the finish line while the toothpaste in the uncoated pipe remains at the start line as shown in FIG. 7E. After a total elapse time of 2.4 seconds, the toothpaste in the coated pipe empties out of the coated pipe while the toothpaste in the uncoated pipe remains at the start line as shown in FIG. 7F.

**Table 1**

| Trial | Time (s) | Velocity (cm/s) |
|---|---|---|
| 1 | 2.21 | 9.05 |
| 2 | 2.56 | 7.81 |
| 3 | 2.54 | 7.87 |
| 4 | 2.84 | 7.04 |
| 5 | 3.38 | 5.92 |

The experiment described in relation to FIGS. 7A-F was conducted five times. The amount of time it took the toothpaste to travel 20 cm from "Start" to "Finish" was measured for each trial. The velocity of the toothpaste was also determined. The toothpaste averaged 7.54 ± 1.16 cm/s in the coated PVC pipe. The toothpaste did not move within the uncoated PVC pipe so the velocity was considered to be zero. The time and velocity of the toothpaste in the coated pipe is shown in Table 1.

*Example 7*

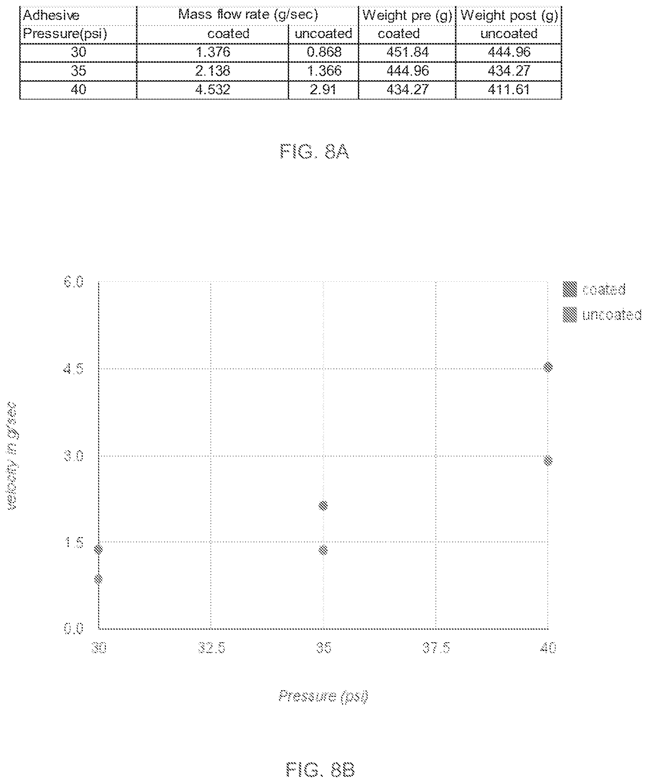

FIGS. 8A-B illustrates the results from an injection molding experiment. Two tubes of construction adhesive (Sonolastic® 150 w/VLM Technology) were obtained and their tips cut to the same ID (7mm) for dispensing. A solution of HF Diblock and Teflon particles (preparation previously described) was sprayed on the inside of the tips and then impregnated with Galden HT200. Each tube was then weighed. Using a pneumatic caulking gun hooked up to a gas cylinder of N2 and a regulator, experiments were performed.

In one experiment, a 6mm diameter drill bit was used to puncture each tube and each was dispensed at three different pressures for a duration of 5 seconds. Timing began as soon as the sealant started exiting the bottle. The pressures were 30, 35, and 40psi respectively. After each dispensing the tubes were weighed and average mass flow rates were calculated as (Mass dispensed)/(Dispense time). The results are summarized in FIGS. 8A-B. The results indicate an increased velocity for the coated nozzle over the standard nozzle of approximately 50% for the same pressure.

Alternatively, for the same mass flow rate, reduced pressure is required for a nozzle with a liquid-impregnated surface at the same mass flow rate than for an uncoated nozzle. For example, in the data provided in the table and graph, the flow rate from a coated nozzle with a pressure of 30 psi as for an uncoated nozzle at 35 psi (1.38 g/s for the coated nozzles compared with 1.37 g/s for the uncoated nozzle) is nearly the same.

This indicates a reduction in power required by 14% (Power required= (Pressure)*(Mass flow rate)/(Density)). The results extend beyond nozzles, but more generally demonstrate that power to flow a material through a tube, pipe, channel, etc, can be significantly reduce the surface the material contacts with has a liquid-impregnated surface.

*Example 8*

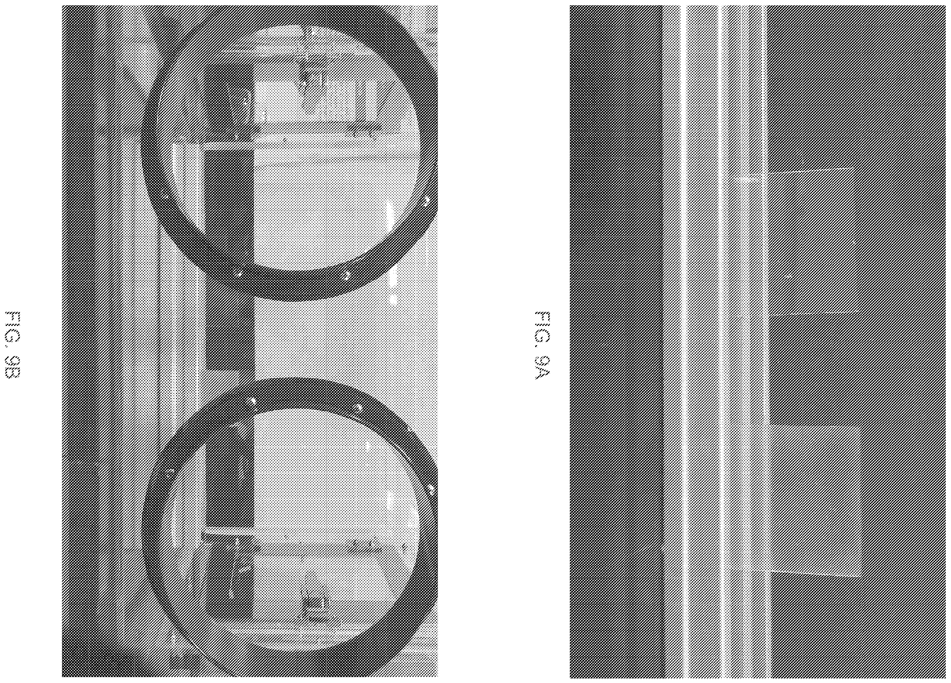

FIGS. 9A-E illustrate a dust capture experiment on liquid-impregnated surfaces. Two roughly 2" squares of PET plastic were cut from a 0.040" thick sheet and weighed. One square was coated with a liquid impregnated surface comprising textured carnauba wax and ethyl oleate and the other was left uncoated as a control. The coated square was then reweighed to account for the coating.

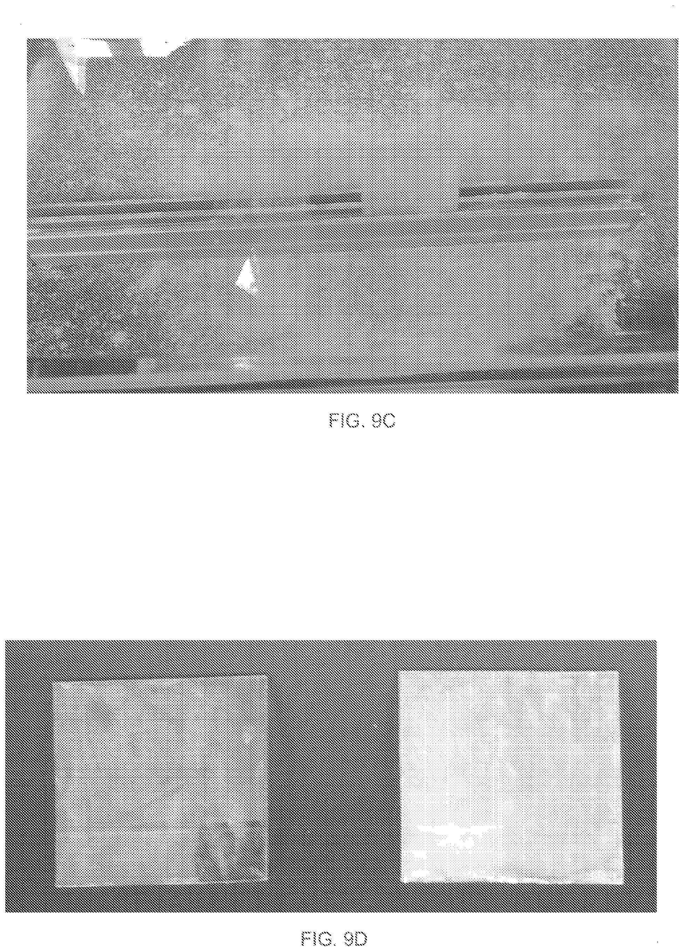

Both squares were place on an aluminum holder within a glove box as shown in FIGS. 9A and 9B. All-purpose flour was then introduced into the glove box as shown in FIG. 9C. Two handfuls were 'clapped' together to create a dust cloud, once in front of the samples and once behind. The parts were taken out of the box as shown in FIG. 9D. The parts were weighed and the weight gain from the powder was calculated to be 0.52 g for on liquid-impregnated surface compared to 0.03 g on the uncoated PET surface. The data is shown in Fig. 9E.

*Example 9*

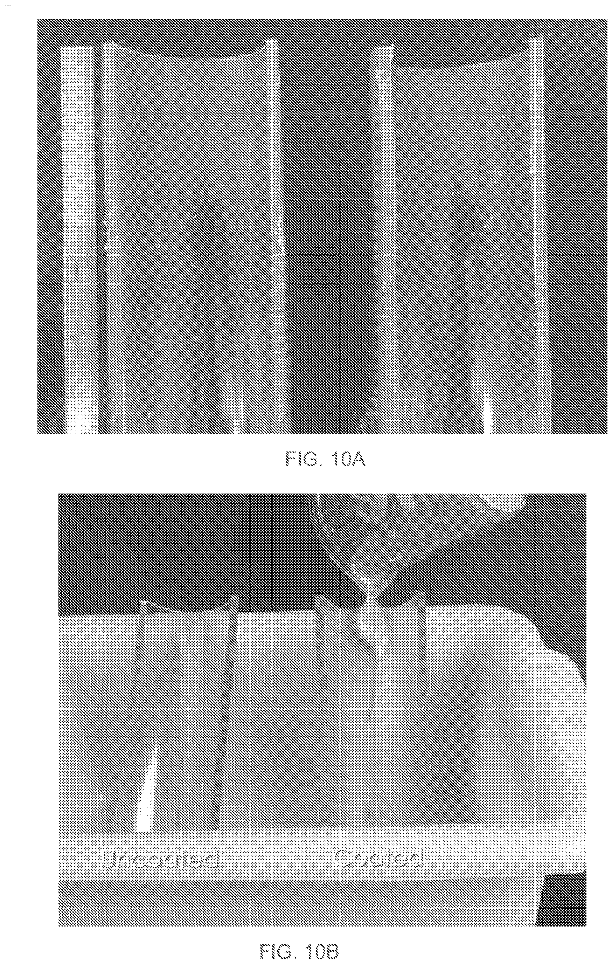

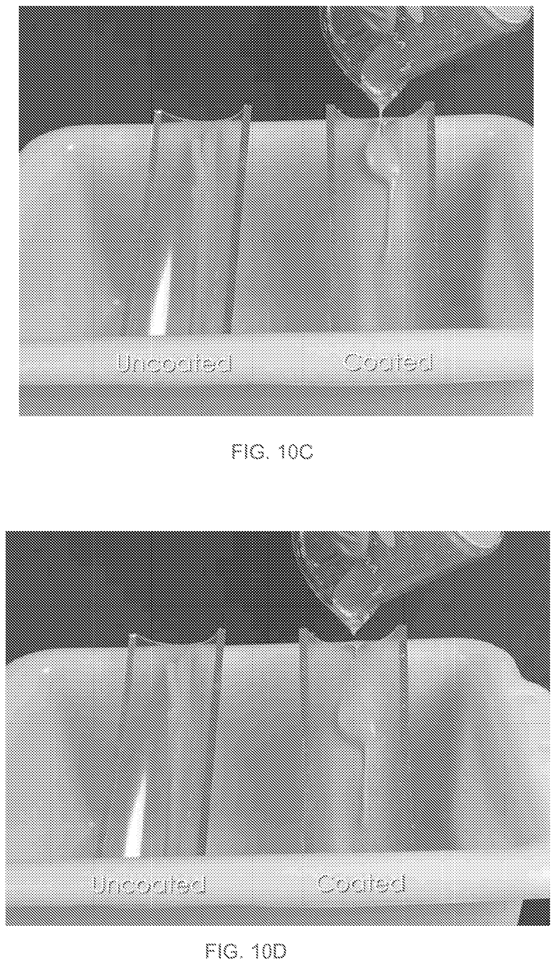

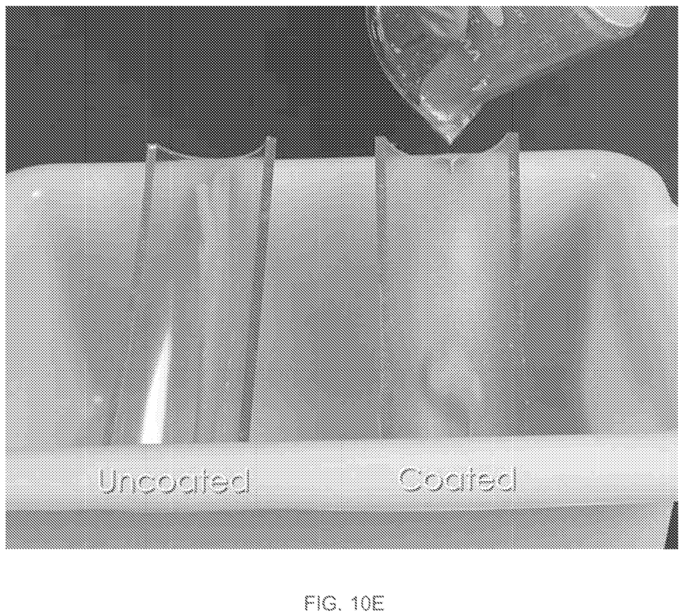

FIGS. 10A-E illustrate a conduit experiment. A 6" long, two-inch diameter PVC pipe was sawn in half lengthwise to create two "conduits" mimicking a chute used for concrete delivery as shown in FIG. 10A. The chute on the right side of FIGS. 10A-E was coated with a liquid-impregnated surface of fluorinated wax and Teflon particles (described elsewhere), and impregnated by Galden HT 200. The chute on the left side of FIGS. 10A-E remained uncoated. The chutes were place side by side in a plastic bucket at a 45°. Quick-set concrete was mixed per the manufacturer's instructions and poured down the uncoated conduit, showing that the uncured concrete sticks to the surface, not all ending up at the bottom as shown in FIGS. 10B-E. The uncured concrete is then poured down the coated conduit. All of the concrete poured down the coated conduit slide off the chute and made it to the bottom of the conduit as shown in FIGS. 10B-E. The concrete applied to the uncoated conduit traveled at a speed of approximately 20 cm/s.

*Example 10*

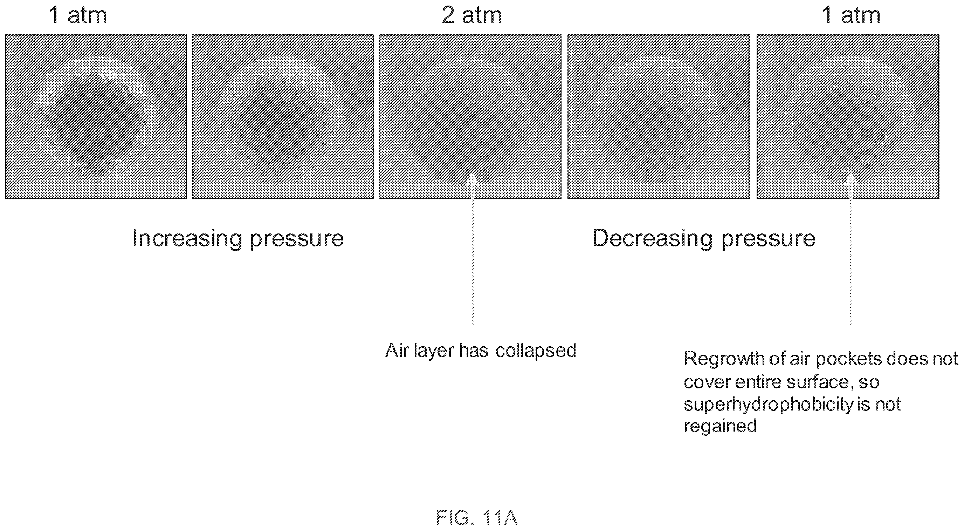

FIGS. 11A-D illustrate an experiment conducted to measure the drag on a metal sphere. Typical super-hydrophobic surfaces involve utilizing a surface roughness on a low-energy surface in order to trap air within the roughness. This air-layer can introduce a velocity-slip with a fluid flowing above the roughness features, as the air is free to move within the air pocket. This ultimately reduces the drag on the above flowing fluid of interest. Yet, as air is easily compressed, this pocket can easily collapse when a pressure/force is introduced. We demonstrate this weakness of superhydrophobic surfaces in FIG. 11A. Here a super hydrophobic aluminum sphere (method of texture creation later described), is submerged in water and pressurized from 1 atm to 2 atm and then depressurized back to 1 atm. The air film completely collapses by 2 atm, and regrows into discrete bubbles after depressurization at 1 atm. Upon collapse the surface is no longer superhydrophobic, and superhydrophobicity is not regained after depressurization. This explains why many superhydrophobic surfaces are not robust for industrial/technical applications. Lubricant-impregnated surfaces involve incorporating a secondary fluid or lubricant, into the surface roughness in place of the air pocket. This lubricant fluid allows for the slip-condition with the primary fluid flowing above it, and is much more stable as liquids are mostly incompressible. The degree of slip can be characterized by linearly extrapolating the velocity profile and, defining a slip length, b, as the depth from the surface at which that profile extrapolates to 0. A balance of shear stresses above and below the interface indicates that.

To estimate the slip length, b, for a liquid-impregnated surface we model the system as shown in Figure.. Linearly extrapolating to zero velocity, we see that the slip length is:

$$b = V_i / (du_x/dy)_o \qquad\qquad \text{Eq. 1}$$

Where $u_x$ is the fluid velocity in the x direction and $V_i$ is the velocity of the oil-water interface, and $(du_x/dy)_o$ is the velocity gradient immediately above the liquid-liquid interface. Recognizing that $V_i = t(du_x/dy)_i$ (where t is the film thickness and $(du_x/dy)_i$ is the velocity gradient within the film), and substituting this into eq. (1), we get:

$$b = t(du_x/dy)_i/(du_x/dy)_o \qquad\qquad \text{Eq. 2}$$

On the top side of the interface, the shear stress equals $\tau_o = \mu_o(du_x/dy)_o$ and on the impregnated liquid side of the interface, $\tau_i = \mu_i(du_x/dy)_i$ Since $\tau_o$ must be equal to $\tau_i$ at the oil-water interface, $\mu_o(du_x/dy)_o = \mu_i(du_x/dy)_i$, Rearranging this gives:

$$\mu_o/\mu_i = (du_x/dy)_i/(du_x/dy)_o \qquad\qquad \text{Eq. 3}$$

Plugging this into equation 2 yields.

$$b = t\,(\mu_o/\mu_i) \quad \text{or} \quad b/t = \mu_o/\mu_i \qquad\qquad \text{Eq. 4}$$

Notice there is practically no benefit to the impregnated surface when $\mu_o/\mu_i < 1$. If this were the case, a larger slip length would be obtained by simply letting the outer liquid fill the texture, rather than impregnating it. (effectively meaning the etched texture lead to reduced drag by simply because the object was smaller in diameter by 2t). If impregnated by a more viscous liquid then the slip length is less than the coating thickness, hence the benefit is as if the solid object were smaller by less than t. This theory predicts the benefit of a liquid-impregnated surface to only be substantial (in the context of reducing drag) when $\mu_o/\mu_i > 1$.

For a sphere falling through a viscous liquid, the terminal velocity it achieves scales as $V_t \sim D^2$, however, a liquid impregnated surface effectively reduces the effective diameter of the sphere by 2b, hence we can more generally write: $V_t \sim (D-2b)^2$.

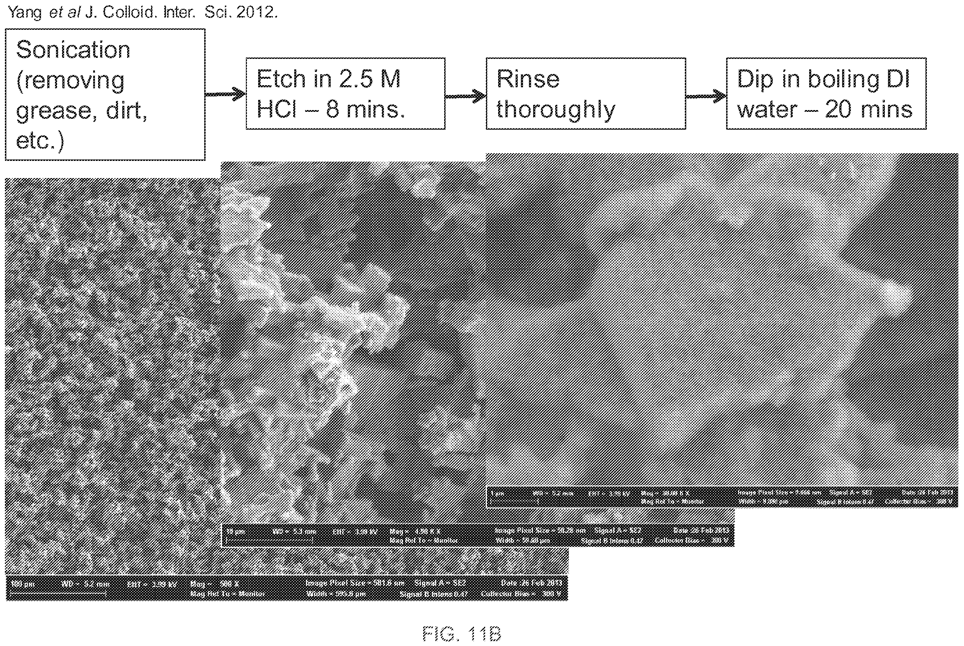

Half-inch diameter aluminum spheres were ultrasonicated with acetone and ethanol to remove dirt/contaminants. Spheres were then etched in a 2.5M HCl solution for ~8 minutes at room temperature. Following the etch, the spheres were first rinsed thoroughly in deionized water and then immersed in boiling deionized water for 20 minutes. Achieved texture on two length scales. The larger roughness is on the order of 5-20 microns and the finer texture on top is on the nanometer scale. Images of this texture are provided in FIG. 11B. From equation 4 we estimate a slip length of approximately 1.1mm for 10 $\mu$m roughness.

Three types of samples were tested. Regular smooth aluminum spheres, super-hydrophobic aluminum spheres, and lubricant-impregnated aluminum spheres, all with 0.5" diameter. Super-hydrophobic spheres were textured with aforementioned texturing process, and then treated with a low-energy silane (octadecyltrichlorosilane-OTS). Lubricant-impregnated samples were textured with the aforementioned texturing process, treated with OTS, and lastly impregnated with a 10 cSt silicone oil by slowly dipping them into reservoirs of the lubricant.

Samples were then carefully dropped into a large bath of glycerin (~1100 cSt). A rectangular container was used to avoid visual distortions of the falling spheres due to the curvature of the container. A pair of sample tweezers was fixed above the container in order to carefully drop the samples directly downward and in the same place each trial. This proved to enhance repeatability of the experiments. Water-glycerin mixtures have been used to vary the viscosity of the bath liquid from pure water (1 cSt) to pure glycerin (~1100 cSt).

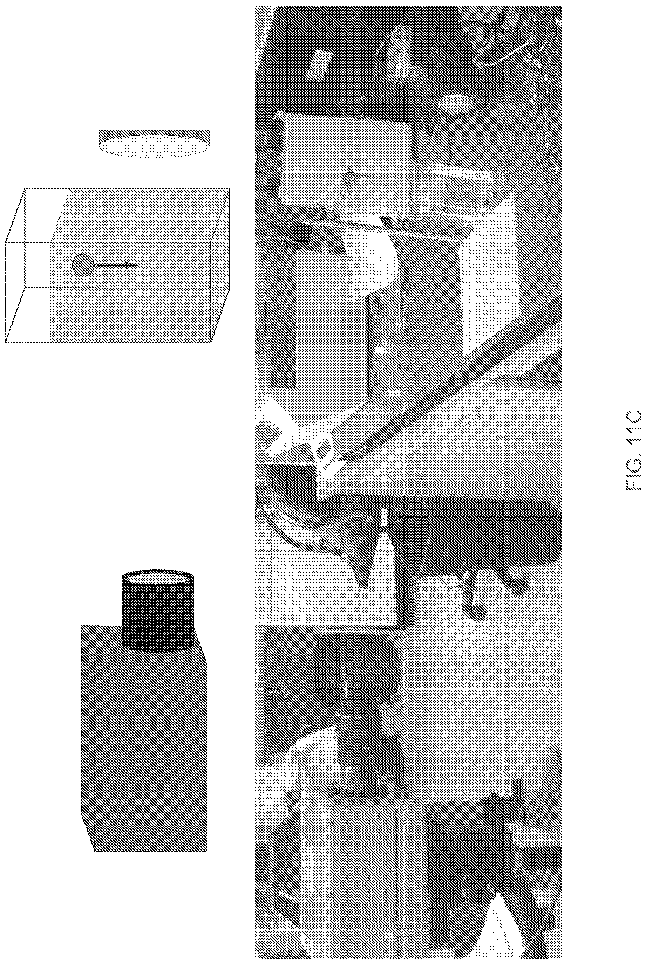

A high-speed camera was used to capture the spheres falling through the bath liquid as shown in FIG. 11C. Once the sphere has reached terminal velocity, the forces of the sphere are in balance (gravity, drag and buoyancy). The spheres in our experiments generally reach constant, terminal velocity within a few centimeters.

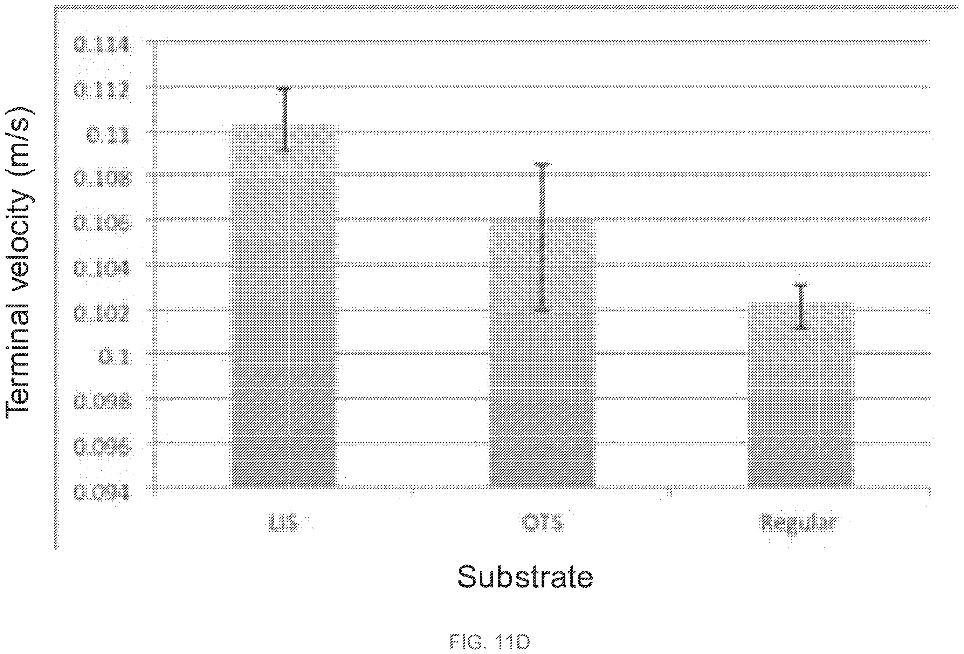

We have been able to show an increase in the terminal velocity (reduction in drag) of the falling aluminum spheres by utilizing an impregnated lubricant in the texture of the spheres as shown in FIG. 11D.

*Example 11*

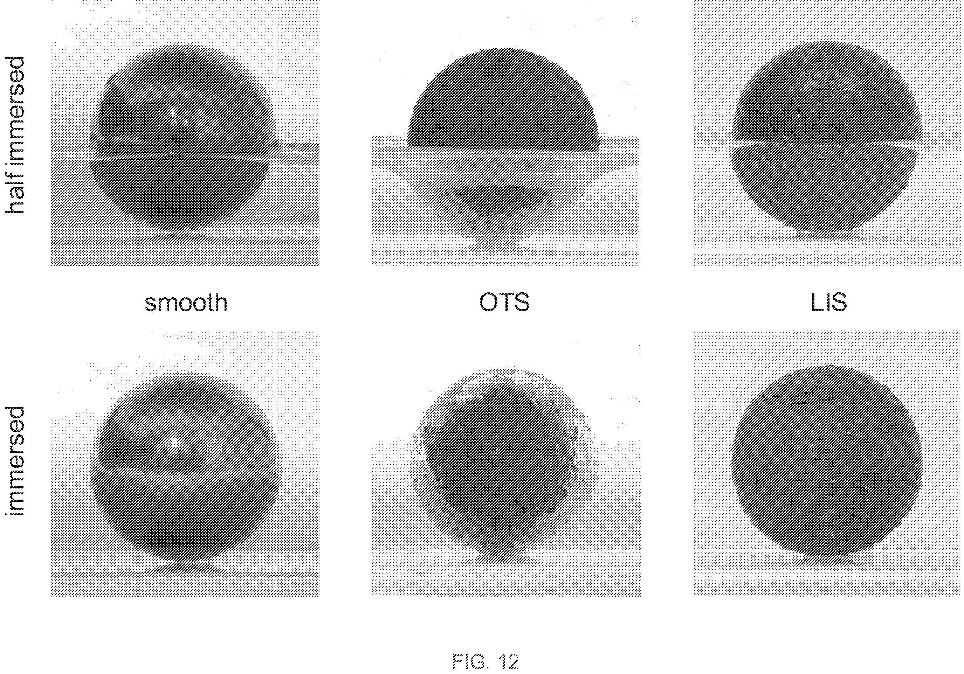

FIG. 12 illustrates two experiments conducted to measure the wetting behavior on a metal sphere. The experiments were conducted with three spheres. The first sphere was a sphere with a smooth surface ("regular"). The second sphere was a sphere with an outer textured surface ("OTS"). The third sphere consisted of a sphere with an liquid-impregnated outer surface ("LTS"). In the first experiment, each sphere was partially submerged in water as shown in the FIG. 12. In the second experiment, each sphere was immersed in water as shown in FIG. 12. The wetting behavior of each sphere is shown in FIG. 12.

*Example 12*

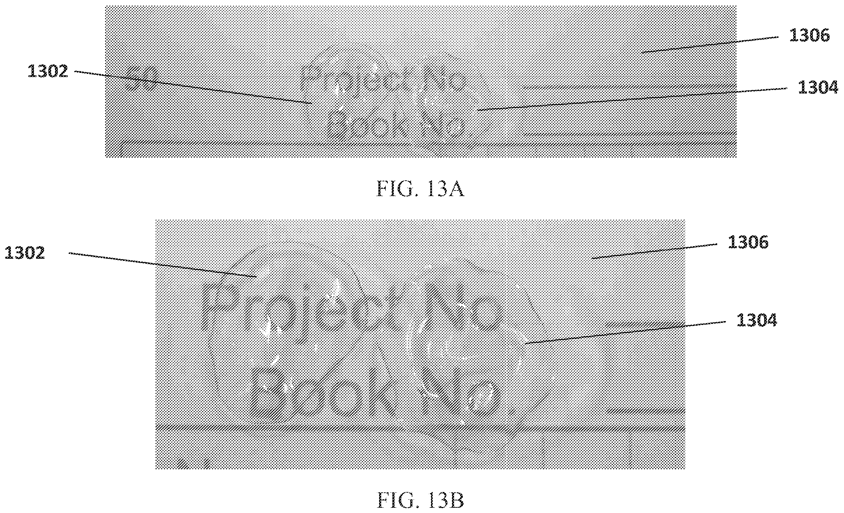

FIGS. 13A and 13B show experimental measurements of transparency of a contact lens with a liquid encapsulated surface when compared to that of a conventional uncoated contact lens.

Two Acuve Oasys contact lenses having a base curve radius of 8.4 millimeters, diameter of 14 millimeters and a power of -0.75 diopters were used for this experiment, labeled lens 1302 and lens 1304. Lenses 1302 and 1304 were dipped in saline solution. Using tweezers, lenses 1302 and 1304 were removed from saline solution and were blow dried with nitrogen gas. Carnuba wax suspension was sprayed onto the interior and exterior surfaces of lens 1304 while holding the lens 1304 at least twelve inches away from the spray nozzle to minimize spray force on the lenses and achieve uniform coating. Subsequently, nitrogen gas was blown across the lens 1304 to allow time to dry coating prior to application of ethyl oleate. Subsequently, ethyl oleate was sprayed onto the interior and exterior surfaces of lens 1304 while holding the lens 1304 at least twelve inches away from the spray nozzle to minimize spray force on the lenses and achieve uniform coating. Finally, contact lenses 1302 and 1304 were placed on notebook page 1306 to provide background and demonstrate transparency of the coating and the photo of FIGS. 13A and 13B was taken. Fig. 13B is a zoomed in image of FIG. 13A.

In this experiment, contact lens 1304 coated with a liquid-impregnated surface comprising carnauba wax and ethyl oleate demonstrated transparency when placed onto a notebook page 206. Words were clearly visible through the transparent coating (See FIGS. 13A and 13B).

Contact angle measurements were performed for both uncoated lens 1302 and coated contact lens 1304. Droplets deposited on the untreated contact lens 1302 were gradually absorbed on the surface indicating that water doesn't slip over the surface. Instead, the deposited water droplets were absorbed. As the contact lens surface of lens 1304 is completely covered by the impregnating liquid-impregnating surface coating, the substrate materials of lens 1304 would not have a substantial effect on the roll-off angles (i.e. the slipperiness) of the surface.

Carnauba wax was applied onto a glass slide and the roll-off angles of a five microliter water droplet on the glass slide was measured to measure the coating performance. The roll-off angle was measured as using a Rame-hart goniometer. The roll-off angle was measured to be approximately 3°. This low roll-off angle demonstrate the ease by which water, which is similar in properties to tear fluid slips over the liquid-impregnated surface.

**Claims**

1. A medical device or medical implement with high lubricity to flesh or biological fluid and/or inhibited nucleation on its surface, the device or implement comprising:

   a surface comprising an impregnating liquid, and
   a plurality of micro-scale solid features spaced sufficiently close to stably contain the impregnating liquid therebetween,
   wherein said impregnating liquid fills spaces between said solid features,
   wherein said surface stably contains said impregnating liquid between said solid features,
   wherein said impregnating liquid is held in place between said plurality of solid features regardless of orientation of said surface,
   wherein the solid features comprise particles having an average dimension in a range of 1 micron to 50 microns; and optionally
   wherein the particles are arranged with average spacing of 1 micron to 30 microns between adjacent particles or clusters of particles.

2. The medical device or medical implement of claim 1, wherein the particles are spray-deposited.

3. The medical device or medical implement of any one of the preceding claims, wherein the impregnating liquid comprises at least one member selected from the group consisting of ethyl oleate, an ester, a fatty acid, a vegetable oil, a terpene, phenyl isothiocyanate, bromobenzene, iodobenzene, o-bromotoluene, alpha-bromonaphthalene, acetylene tetrabromide, 1-butyl-3-methylimidazolium bis(trifluoromethylsulfonyl) imide (BMIm), tribromohydrin (1,2,3-tribromopropane), ethylene dibromide, carbon disulfide, bromoform, methylene iodide (diiodomethane), p-bromotoluene, perchloroethylene, alpha-monochloro-naphthalene, aniline, butyl alcohol, isoamyl alcohol, n-heptyl alcohol, cresol, oleic acid, linoleic acid, amyl phthalate and perfluorinated polyethers.

4. The medical device or medical implement of any one of the preceding claims, wherein the solid features comprise one or more members selected from the group consisting of wax, carnauba wax, beeswax, candelilla wax, zein, dextrin, cellulose ether, hydroxyethyl cellulose, hydroxypropyl cellulose (HPC), hydroxyethyl methyl cellulose, hydroxypropyl methyl cellulose (HPMC), ethyl hydroxyethyl cellulose, purified wood cellulose, micro-crystalline cellulose, kaolinite, Japan wax, pulp (e.g., spongy part of plant stems), ferric oxide, iron oxide, sodium formate, sodium oleate, sodium palmitate, sodium sulfate, a metal, a polymer, a ceramic solid, a fluorinated solid, an intermetallic solid, and a composite solid PDMS, cyclic olefin polymer, polypropylene, PVC, PET, HDPE, polyimide, PMMA, glass, Perspex™, Plexiglass™, polymacon.

5. The medical device or medical implement of any one of the preceding claims, wherein the impregnating liquid comprises an additive to prevent or reduce evaporation of the impregnating liquid.

6. The medical device or medical implement of any one of the preceding claims, wherein the medical device or medical implement is a member selected from the group consisting of braces, dentures, a retainer, orthodonture, a bridge, an implant, a tooth/teeth mold, a prosthesis, an artificial organ, an artificial artery, a stent, a syringe, a lining (e.g., lining for artery walls to prevent plaque formation), an IV tube, an IV bag, a colostomy bag, a surgical instrument, a bandage, and a blood pump.

7. The medical device or medical implement of any one of the preceding claims, wherein said medical device or medical implement is a blood pump or part thereof, wherein the surface is configured to provide reduction of shear forces to prevent damage to cells and/or other biological structures in blood or other biological fluids being pumped thereby or therethrough.

8. The medical device or medical implement of any one of the preceding claims, wherein said medical device or medical implement is a member selected from the group consisting of a pill, capsule (e.g., single-piece or two-piece), tablet, gel cap, and suppository.

9. The medical device or medical implement of any one of the preceding claims, wherein said medical device or medical implement is a member selected from the group consisting of a micropipette, a small volume container of biological material, a human serum container, a pipette, a pipette tip, a microfluidic device, a dialysis machine, a tube, an endoscope, an intubation device, a syringe, a stent, a catheter, and a tracheotomy tube.

10. The medical device or medical implement of any one of the preceding claims, wherein said medical device or medical implement is a member selected from the group consisting of a glove, bandage, adhesive strip, drug release patch, and condom.

11. The medical device or medical implement of any one of the preceding claims, wherein said impregnating liquid:

    a) comprises a drug or bioactive components; and/or
    b) is curable and can be converted to a solid by curing.

12. The medical device or medical implement of any one of the preceding claims, wherein
$S_{ow(a)} < 0$, where $S_{ow(a)}$ is spreading coefficient, defined as $\gamma_{wa} - \gamma_{wo} - \gamma_{oa}\, \gamma$, where $\gamma$ is the interfacial tension between the two phases designated by subscripts w, a, and o, where w is water, a is air, and o is the impregnating liquid.

13. The medical device or medical implement of any one of claims 1 to 12, wherein $0.01 < \phi \leq 0.10$, where $\phi$ is a representative fraction of the projected surface area of the liquid-impregnated surface corresponding to non-submerged solid at equilibrium.

14. The medical device or medical implement of any one of claims 1 to 11, wherein one or both of the following holds:

    (i) $\theta_{os(w),receding} = 0$; and
    (ii) $\theta_{os(a),receding} = 0$ and $\theta_{os(w),receding} = 0$,

where $\theta_{os(w),receding}$ receding is receding contact angle of the impregnating liquid (e.g., oil, subscript 'o') on the surface (subscript 's') in the presence of water (subscript 'w'), and where $\theta_{os(w),receding}$ is receding contact angle of the impregnating liquid (e.g., oil, subscript 'o') on the surface (subscript 's') in the presence of air (subscript 'a').

**Patentansprüche**

1. Medizinische Vorrichtung oder medizinisches Gerät mit einer hohen Gleitfähigkeit gegenüber Fleisch oder biologischem Fluid und/oder gehemmter Keimbildung auf ihrer/seiner Oberfläche, wobei die Vorrichtung oder das Gerät umfasst:

eine Oberfläche, die eine imprägnierende Flüssigkeit umfasst, und
mehrere Festkörpergebilde im Mikromaßstab, die ausreichend eng beabstandet sind, um die imprägnierende Flüssigkeit dazwischen stabil zu enthalten,
wobei die imprägnierende Flüssigkeit Räume zwischen den Festkörpergebilden füllt,
wobei die Oberfläche die imprägnierende Flüssigkeit zwischen den Festkörpergebilden stabil enthält,
wobei die imprägnierende Flüssigkeit zwischen den mehreren Festkörpergebilden unabhängig von der Ausrichtung der Oberfläche festgehalten wird,
wobei die Festkörpergebilde Partikel mit einer durchschnittlichen Abmessung in einem Bereich von 1 Mikrometer bis 50 Mikrometer umfasst, und
wobei die Partikel optional mit einem durchschnittlichen Abstand von 1 Mikrometer bis 30 Mikrometer zwischen benachbarten Partikeln oder Partikelclustern angeordnet sind.

2.  Medizinische Vorrichtung oder medizinisches Gerät gemäß Anspruch 1,
    wobei die Partikel aufgesprüht sind.

3.  Medizinische Vorrichtung oder medizinisches Gerät gemäß einem der vorhergehenden Ansprüche, wobei die imprägnierende Flüssigkeit mindestens ein Element, ausgewählt aus der Gruppe, bestehend aus Ethyloleat, einem Ester, einer Fettsäure, einem pflanzlichen Öl, einem Terpen, Phenylisothiocyanat, Brombenzol, Iodbenzol, o-Bromtoluol, alpha-Bromnaphthalin, Acetylentetrabromid, 1-Butyl-3-methylimidazoliumbis(trifluormethylsulfonylimid (BMIm), Triborhydrin-(1,2,3-tribrompropan), Ethylendibromid, Kohlenstoffdisulfid, Bromoform, Methyleniodid (Diiodmethan), p-Bromtoluol, Perchlorethylen, alpha-Monochlor-Naphthalin, Anilin, Butylalkohol, Isoamylalkohol, n-Heptylalkohol, Cresol, Ölsäure, Linolsäure, Amylphthalat und perfluorierten Polyethern, umfasst.

4.  Medizinische Vorrichtung oder medizinisches Gerät gemäß einem der vorhergehenden Ansprüche, wobei die Festkörpergebilde ein oder mehrere Elemente, ausgewählt aus der Gruppe, bestehend aus Wachs, Karnaubawachs, Bienenwachs, Candelillawachs, Zein, Dextrin, Celluloseether, Hydroxyethylcellulose, Hydroxypropylcellulose (HPC), Hydroxyethylmethylcellulose, Hydroxypropylmethylcellulose (HPMC), Ethylhydroxyethylcellulose, gereinigter Holzcellulose, mikrokristalline Cellulose, Kaolinit, Japanwachs, Zellstoff (z.B. spongiöser Teil von Pflanzenstämmen), Eisen(III)-Oxid, Eisenoxid, Natriumformiat, Natriumoleat, Natriumpalmitat, Natriumsulfat, einem Metall, einem Polymer, einem keramischen Feststoff, einem fluorierten Feststoff, einem intermetallischen Feststoff und einem zusammengesetzten Feststoff, PDMS, cyclischem Olefinpolymer, Polypropylen, PVC, PET, HDPE, Polyimid, PMMA, Glas, Perspex™, Plexiglas™, Polymacon, umfasst.

5.  Medizinische Vorrichtung oder medizinisches Gerät gemäß einem der vorhergehenden Ansprüche, wobei die imprägnierende Flüssigkeit ein Additiv umfasst, um die Verdampfung der imprägnierenden Flüssigkeit zu verhindern oder zu reduzieren.

6.  Medizinische Vorrichtung oder medizinisches Gerät gemäß einem der vorhergehenden Ansprüche, wobei die medizinische Vorrichtung oder das medizinische Gerät ein Element, ausgewählt aus der Gruppe, bestehend aus Zahnspangen, Zahlersatz, Retainer, Kieferorthopädie, einer Brücke, eines Implantats, einer Zahn/Zähne-Abgussform, einer Prothese, eines künstlichen Organs, einer künstlichen Arterie, eines Stents, einer Spritze, einer Auskleidung (z.B. Auskleidung für Arterienwände, um Plaquebildung zu verhindern), eines IV-Schlauchs, eines IV-Beutels, eines Kolostomiebeutels, eines chirurgischen Instruments, einer Bandage und einer Blutpumpe, ist.

7.  Medizinische Vorrichtung oder medizinisches Gerät gemäß einem der vorhergehenden Ansprüche, wobei die medizinische Vorrichtung oder das medizinische Gerät eine Blutpumpe oder ein Teil davon ist, wobei die Oberfläche so gestaltet ist, dass sie eine Verringerung der Scherkräfte ermöglicht, um eine Schädigung von Zellen und/oder anderen biologischen Strukturen im Blut oder anderen biologischen Fluiden, die durch sie oder durch sie hindurch gepumpt werden, zu verhindern.

8.  Medizinische Vorrichtung oder medizinisches Gerät gemäß einem der vorhergehenden Ansprüche, wobei die medizinische Vorrichtung oder das medizinische Gerät ein Element, ausgewählt aus der Gruppe, bestehend aus einer Pille, Kapsel (z.B. einstückig oder zweistückig), Tablette, Gelkapsel oder einem Suppositorium, ist.

9.  Medizinische Vorrichtung oder medizinisches Gerät gemäß einem der vorhergehenden Ansprüche, wobei die medizinische Vorrichtung oder das medizinische Gerät ein Element, ausgewählt aus der Gruppe, bestehend aus einer Mikropipette, einem kleinvolumigen Behälter für biologisches Material, einen Behälter für menschliches Serum, einer Pipette, einer Pipettenspitze, einer Mikrofluidvorrichtung, einem Dialysegerät, einem Schlauch, einem Endoskop, einer Intubationsvorrichtung, einer Spritze, einem Stunt, einem Katheter und einer Tracheotomiekanüle, ist.

10. Medizinische Vorrichtung oder medizinisches Gerät gemäß einem der vorhergehenden Ansprüche, wobei die medizinische Vorrichtung oder das medizinische Gerät ein Element, ausgewählt aus der Gruppe, bestehend aus einem Handschuh, einer Bandage, einem Haftstreifen, einem Arzneimittel-freisetzenden Pflaster und einem Kondom, ist.

11. Medizinische Vorrichtung oder medizinisches Gerät gemäß einem der vorhergehenden Ansprüche, wobei die imprägnierende Flüssigkeit:

> a) ein Arzneimittel oder bioaktive Komponenten umfasst und/oder
> b) härtbar ist und durch Härtung in einen Feststoff umgewandelt werden kann.

12. Medizinische Vorrichtung oder medizinisches Gerät gemäß einem der vorhergehenden Ansprüche, wobei $S_{ow(a)} < 0$ ist, worin $S_{ow(a)}$ ein Spreitkoeffizient, definiert als $\gamma_{wa} - \gamma_{wo} - \gamma_{oa}$, ist, wobei $\gamma$ die Grenzflächenspannung zwischen zwei Phasen, die durch die Indizes w, a und o gekennzeichnet sind, wobei w Wasser ist, a Luft ist und o die imprägnierende Flüssigkeit ist.

13. Medizinische Vorrichtung oder medizinisches Gerät gemäß einem der Ansprüche 1 bis 12, wobei $0,01 < \phi \leq 0,10$ ist, worin $\phi$ ein repräsentativer Anteil der projizierten Oberfläche der mit der Flüssigkeit imprägnierten Oberfläche, entsprechend dem nicht eingetauchtem Festkörper im Gleichgewicht, ist.

14. Medizinische Vorrichtung oder medizinisches Gerät gemäß einem der Ansprüche 1 bis 11, wobei eines oder beide der folgenden gilt:

> (i) $\theta_{os(w)}$, *Rückzug* = 0 und
> (ii) $\theta_{os(a)}$, *Rückzug* = 0 und $\theta_{os(w)}$, *Rückzug* = 0,

worin $\theta_{os(w)}$, *Rückzug* ein Rückzugskontaktwinkel der imprägnierenden Flüssigkeit (z.B. Öl, Index "o") auf der Oberfläche (Index "s") in Gegenwart von Wasser (Index "w") ist und wobei $\theta_{os(w)}$, *Rückzug* ein Rückzugswinkel der imprägnierenden Flüssigkeit (z.B. Öl, Index "o") auf der Oberfläche (Index "s") in Gegenwart von Luft (Index "a") ist.

## Revendications

1. Dispositif médical ou instrument médical comportant un haut pouvoir lubrifiant sur la chair ou un fluide biologique et/ou une nucléation inhibée à sa surface, le dispositif ou l'instrument comprenant :

> une surface comprenant un liquide d'imprégnation, et
> une pluralité de caractéristiques solides à micro-échelle espacées de manière suffisamment proche les unes par rapport aux autres pour contenir de manière stable le liquide d'imprégnation entre elles,
> dans lequel ledit liquide d'imprégnation remplit des espaces entres lesdites caractéristiques solides,
> dans lequel ladite surface contient de manière stable ledit liquide d'imprégnation entre lesdites caractéristiques solides,
> dans lequel ledit liquide d'imprégnation est maintenu en place entre ladite pluralité de caractéristiques solides quelle que soit l'orientation de ladite surface,
> dans lequel les caractéristiques solides comprennent des particules présentant une dimension moyenne située dans une plage allant de 1 micromètre à 50 micromètres ; et facultativement
> dans lequel les particules sont agencées avec un espacement moyen allant de 1 micromètre à 30 micromètres entre des particules adjacentes ou des amas de particules.

2. Dispositif médical ou instrument médical selon la revendication 1, dans lequel les particules sont déposées par pulvérisation.

3. Dispositif médical ou instrument médical selon l'une quelconque des revendications précédentes, dans lequel le liquide d'imprégnation comprend au moins un élément choisi parmi le groupe consistant en oléate d'éthyle, un ester, un acide gras, une huile végétale, un terpène, isothiocyanate de phényle, bromobenzène, iodobenzène, o-bromotoluène, alpha-bromonaphthalène, tétrabromure d'acétylène, 1-butyl-3-méthylimidazolium bis(trifluorométhylsulfonyl)imide (BMIm), tribromohydrin (1,2,3-tribromopropane), dibromure d'éthylène, disulfure de carbone, bromoforme, iodure de méthylène (diiodométhane), p-bromotoluène, perchloroéthylène, alpha-monochloronaphthalène, aniline, alcool butylique, alcool isoamylique, alcool n-heptylique, crésol, acide oléique, acide linoléique, phtalate d'amyle et polyéthers perfluorés.

4. Dispositif médical ou instrument médical selon l'une quelconque des revendications précédentes, dans lequel les caractéristiques solides comprennent un ou plusieurs éléments choisis parmi le groupe consistant en cire, cire de carnauba, cire d'abeille, cire de Candelilla, zéine, dextrine, éther de cellulose, hydroxyéthylcellulose, hydroxypropylcellulose (HPC), hydroxyéthylméthylcellulose, hydroxypropylméthylcellulose (HPMC), éthylhydroxyéthylcellulose, cellulose de bois purifiée, cellulose microcristalline, kaolinite, cire du Japon, pulpe (p. ex., partie spongieuse des tiges des plantes), oxyde ferrique, oxyde de fer, formiate de sodium, oléate de sodium, palmitate de sodium, sulfate de sodium, un métal, un polymère, un solide en céramique, un solide fluoré, un solide intermétallique, et un solide composite PDMS, polymère d'oléfine cyclique, polypropylène, PVC, PET, PEHD, polyimide, PMMA, verre, Perspex™, Plexiglass™, polymacon.

5. Dispositif médical ou instrument médical selon l'une quelconque des revendications précédentes, dans lequel le liquide d'imprégnation comprend un additif pour empêcher ou diminuer l'évaporation du liquide d'imprégnation.

6. Dispositif médical ou instrument médical selon l'une quelconque des revendications précédentes, dans lequel le dispositif médical ou l'instrument médical est un élément choisi parmi le groupe consistant en appareil dentaire, prothèse dentaire, dispositif de retenue, orthodontie, un bridge, un implant, un moule de dent/dents, une prothèse, un organe artificiel, une artère artificielle, une endoprothèse, une seringue, un revêtement (p. ex. un revêtement pour les parois des artères permettant d'empêcher la formation de plaque), un tube IV, une poche IV, une poche de colostomie, un instrument chirurgical, un pansement et une pompe à sang.

7. Dispositif médical ou instrument médical selon l'une quelconque des revendications précédentes, dans lequel ledit dispositif médical ou instrument médical est une pompe à sang ou une partie de celle-ci, dans lequel la surface est configurée pour réduire les forces de cisaillement afin d'empêcher de léser les cellules et/ou d'autres structures biologiques dans le sang ou d'autres fluides biologiques étant pompés par celle-ci ou à travers celle-ci.

8. Dispositif médical ou instrument médical selon l'une quelconque des revendications précédentes, dans lequel ledit dispositif médical ou instrument médical est un élément choisi parmi le groupe consistant en une pilule, une gélule (p. ex., en une partie ou en deux parties), un comprimé, une capsule molle et un suppositoire.

9. Dispositif médical ou instrument médical selon l'une quelconque des revendications précédentes, dans lequel ledit dispositif médical ou instrument médical est un élément choisi parmi le groupe consistant en une micropipette, un récipient de petit volume contenant du matériel biologique, un récipient de sérum humain, une pipette, une pointe de pipette, un dispositif microfluidique, un dialyseur, un tube, un endoscope, un dispositif d'intubation, une seringue, une endoprothèse et un tube de trachéotomie.

10. Dispositif médical ou instrument médical selon l'une quelconque des revendications précédentes, dans lequel ledit dispositif médical ou instrument médical est un élément choisi parmi le groupe consistant en un gant, un pansement, une bande adhésive, un timbre transdermique et un préservatif.

11. Dispositif médical ou instrument médical selon l'une quelconque des revendications précédentes, dans lequel ledit liquide d'imprégnation :

    a) comprend un médicament ou des composants bioactifs ; et/ou
    b) est durcissable et peut être converti en un solide par durcissement.

12. Dispositif médical ou instrument médical selon l'une quelconque des revendications précédentes, dans lequel Sow(a) < 0, où Sow(a) est le coefficient de diffusion, défini tel que $\gamma_{wa} - \gamma_{wo} - \gamma_{oa}\ \gamma$, où $\gamma$ est la tension interfaciale entre les deux phases désignée par des indices w, a et o, où w représente l'eau, a représente l'air et o représente le liquide d'imprégnation.

13. Dispositif médical ou instrument médical selon l'une quelconque des revendications 1 à 12, dans lequel $0{,}01 < \phi \leq$

0,10, où $\Phi$ est une fraction représentative de la zone de surface projetée de la surface imprégnée de liquide correspondant au solide non immergé à l'équilibre.

14. Dispositif médical ou instrument médical selon l'une quelconque des revendications 1 à 11, dans lequel l'une ou les deux des conditions suivantes sont remplies :

(i) $\theta_{os(w),recul} = 0$; et
(ii) $\theta_{os(a),recul} = 0$ et $\theta_{os(w)} = 0$,

où $\theta_{os(w),recul}$ recul désigne l'angle de contact de recul du liquide d'imprégnation (p. ex., huile, indice 'o') à la surface (indice 's') en présence d'eau (indice 'w'), et où $\theta_{os(w),recul}$ désigne l'angle de contact de recul du liquide imprégné (p. ex., huile, indice 'o') à la surface (indice 's') en présence d'air (indice 'a').

EP 2 855 603 B1

FIG. 1

FIG. 2B

FIG. 2A

Coated   Uncoated

304
308
302
306

FIG. 3A

Coated   Uncoated

308
304
306
302

FIG. 3B

FIG. 4A

FIG. 4B

FIG. 5A

FIG. 5B

FIG. 5C

FIG. 5D

# Adhesion strength calculations

N

glass slide →

Liquid-impregnated surface (2x2 cm)

8888

scale ⟶

Average measurement: 44.85 ± 4 g

$$\tau_{adh} = \frac{N}{A}$$

N = 4.4E-4 ± 4E-5 N

A = 0.004 m$^{-2}$

$\tau_{adh}$ = 1.1 ± 0.1 Pa

The adhesion force was obtained by measuring the force needed to separate a liquid-impregnated surface from a glass slide in the normal direction. A glass slide was attached to the scale and the liquid-impregnated surface was pulled off of the surface in the normal direction. Capillarity forces due to the impregnated liquid resulted in adhesive strength of $\tau_{adh}$= 1.1± 0.1 Pa.

Preparation of the liquid-impregnated surface: The surface was comprised of a lithography patterned array of square posts of 10 um width and height, and spaced by 25 um. 10 cSt silicone oil was impregnated into the surface.

FIG. 6

EP 2 855 603 B1

FIG. 7A

FIG. 7B

FIG. 7C

FIG. 7D

FIG. 7E

FIG. 7F

| Adhesive | Mass flow rate (g/sec) | | Weight pre (g) | Weight post (g) |
|---|---|---|---|---|
| Pressure(psi) | coated | uncoated | coated | uncoated |
| 30 | 1.376 | 0.868 | 451.84 | 444.96 |
| 35 | 2.138 | 1.366 | 444.96 | 434.27 |
| 40 | 4.532 | 2.91 | 434.27 | 411.61 |

FIG. 8A

FIG. 8B

FIG. 9A

FIG. 9B

FIG. 9C

FIG. 9D

FIG. 10A

FIG. 10B

FIG. 10C

FIG. 10D

FIG. 10E

FIG. 11A

Yang *et al* J. Colloid. Inter. Sci. 2012.

Sonication (removing grease, dirt, etc.) → Etch in 2.5 M HCl – 8 mins. → Rinse thoroughly → Dip in boiling DI water – 20 mins

FIG. 11B

FIG. 11C

FIG. 11D

FIG. 12

FIG. 13A

FIG. 13B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 03071275 A
- WO 2010129807 A
- US 302356
- US 20130032316 A, Smith
- US 517552, Smith
- US 61827444, Smith

### Non-patent literature cited in the description

- **STAUFFER, C. E.** The measurement of surface tension by the pendant drop technique. *J. Phys. Chem.,* 1965, vol. 69, 1933-1938